(19) Europäisches Patentamt / European Patent Office / Office européen des brevets

(11) **EP 3 897 827 B1**

(12) **EUROPÄISCHE PATENTSCHRIFT**

(45) Veröffentlichungstag und Bekanntmachung des Hinweises auf die Patenterteilung:
**24.07.2024   Patentblatt 2024/30**

(51) Internationale Patentklassifikation (IPC):
*A61N 5/06* (2006.01)     *A61N 5/067* (2006.01)
*A61B 18/22* (2006.01)     *G02B 5/02* (2006.01)
*C03C 3/089* (2006.01)     *C03C 3/093* (2006.01)
*C03C 3/105* (2006.01)     *C03C 14/00* (2006.01)
*G02B 6/26* (2006.01)

(21) Anmeldenummer: **19829555.2**

(22) Anmeldetag: **19.12.2019**

(52) Gemeinsame Patentklassifikation (CPC):
**C03C 3/093; A61B 18/22; A61N 5/0601;**
**A61N 5/062; A61N 5/0625; A61N 5/067;**
**C03C 3/089; C03C 3/105; C03C 14/008;**
**G02B 5/0247; G02B 5/0268;** A61B 2018/2261;
A61N 2005/0602; A61N 2005/063; C03C 2214/08;

(Forts.)

(86) Internationale Anmeldenummer:
**PCT/EP2019/086345**

(87) Internationale Veröffentlichungsnummer:
**WO 2020/127762 (25.06.2020 Gazette 2020/26)**

(54) **BELEUCHTUNGSSYSTEM MIT EINEM LICHTLEITER MIT IM WESENTLICHEN RADIAL ABSTRAHLENDEM DIFFUSOR-ELEMENT SOWIE VERFAHREN ZU DESSEN HERSTELLUNG**

ILLUMINATION SYSTEM HAVING AN OPTICAL WAVEGUIDE WITH SUBSTANTIALLY RADIALLY EMITTING DIFFUSER ELEMENT, AND METHOD FOR PRODUCTION THEREOF

SYSTÈME D'ÉCLAIRAGE COMPRENANT UN CONDUCTEUR DE LUMIÈRE COMPRENANT UN ÉLÉMENT DIFFUSEUR RAYONNANT ESSENTIELLEMENT RADIALEMENT AINSI QUE PROCÉDÉ POUR LA FABRICATION DE CELUI-CI

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.12.2018   DE 102018133338**

(43) Veröffentlichungstag der Anmeldung:
**27.10.2021   Patentblatt 2021/43**

(73) Patentinhaber: **SCHOTT AG**
**55122 Mainz (DE)**

(72) Erfinder:
• **SCHULTHEIS, Bernd**
**55270 Schwabenheim (DE)**
• **KEIPER, Oliver**
**65510 Hünstetten (DE)**

• **MEINL, Jürgen**
**65329 Hohenstein-Holzhausen (DE)**
• **RUSSERT, Hubertus**
**55270 Jugenheim (DE)**
• **GRIMM, Jonas**
**65307 Bad Schwalbach (DE)**
• **WILLMES, Lothar**
**65375 Oestrich-Winkel (DE)**
• **CRAMER, Martin**
**65187 Wiesbaden (DE)**

(74) Vertreter: **Blumbach · Zinngrebe Patentanwälte PartG mbB**
**Alexandrastraße 5**
**65187 Wiesbaden (DE)**

(56) Entgegenhaltungen:
**EP-A1- 3 184 885       WO-A1-2017/103796**
**US-A1- 2014 268 815     US-A1- 2014 270 639**

(52) Gemeinsame Patentklassifikation (CPC): (Forts.)
C03C 2214/16; C03C 2214/20; G02B 6/262

**Beschreibung**

Die Erfindung betrifft ein Beleuchtungssystem, insbesondere für ein medizintechnisches Therapie- und/oder Diagnosesystem, umfassend wenigstens eine Laser-Lichtquelle, und einen Lichtleiter, der an einem proximalen Ende an die wenigstens eine Laser-Lichtquelle anschließbar und/oder dieser zuordenbar ist, und welches am distalen Ende des Lichtleiters ein Diffusor-Element umfasst. Die Erfindung betrifft ferner ein Verfahren zur Herstellung des Beleuchtungssystems.

Derartige Beleuchtungssysteme kommen verstärkt im medizinischen Umfeld zum Einsatz. Dabei lassen sich folgende Anwendungsschwerpunkte klassifizieren:

- Photodynamische Therapie (PDT) und/oder Photo-Immuno-Therapie (PIT) zur Tumortherapie
- Endovenöse Lasertherapie (EVLT) zur Behandlung von Krampfadern
- Laser induzierte interstitielle Thermotherapie (LITT) sowie
- sonstige Anwendungen, u.a. im Bereich der Dentalmedizin, Augenheilkunde sowie Dermatologie.

Die Photodynamische Therapie (PDT) ist eine minimalinvasive Therapiemöglichkeit bei verschiedenen Krebserkrankungen. Unter der PDT versteht man ein Verfahren zur Behandlung von Tumoren und anderen Gewebeveränderungen (wie beispielsweise Gefäßneubildungen) mit Licht in Kombination mit einer lichtaktivierbaren Substanz. Zu Beginn der Behandlung werden den Patienten intravenös lichtsensible Substanzen, sogenannte Photosensitizer, in die Blutbahn injiziert, die sich in beziehungsweise an den Krebszellen anreichern. Diese natürlichen Photosubstanzen konzentrieren sich in den Tumorzellen und bewirken dort eine starke Lichtempfindlichkeit. Dazu können während der PDT-Behandlung in das Tumor-Gewebe mehrere Kanülen (typ. bis zu 8) gestochen, in die jeweils ein Lichtleiter mit einem Diffusor-Element eingeführt wird, wobei die Diffusor-Elemente möglichst räumlich über das Tumor-Gewebe verteilt angeordnet sein müssen. Laser-Licht, in der Regel mit Wellenlängen im sichtbaren Spektralbereich, zum Beispiel Grün-Licht mit 532 nm oder Rot-Licht mit 690 nm Wellenlänge, wird über die Lichtleiter in die Diffusor-Elemente eingekoppelt, so dass das Tumor-Gewebe möglichst gleichmäßig von innen ausgeleuchtet wird. Dabei bilden sich in diesen Zellen aggressive Sauerstoffradikale, welche die Tumorzellen selektiv zerstören. Im Gegensatz zu den kranken Zellen bleiben die gesunden Zellen von dieser chemischen Reaktion unberührt. Der genaue Wirkmechanismus ist u.a. in "Photodynamic Therapy of Cancer", Cancer Medicine, 2003 beschrieben.

Bei der Photo-Immuno-Therapie (PIT) wird der Photosensitizer an einen Antikörper gebunden, damit das Arzneimittel selektiver an das Tumorgewebe abgegeben werden kann. Der Antikörper ist spezifisch gegen ein Protein, das auf der Oberfläche des Tumors vorhanden ist, so dass er spezifisch den Tumor ansteuert. Dann wird der Tumor mit Licht bestrahlt, wodurch dessen Zerstörung ausgelöst wird. Idealerweise erreicht der am Antikörper gebundene Photosensitizer die empfindlichsten Teile der Krebszelle wie z. B. die Lysosomen, die Verdauungsenzym enthalten. Die Zerstörung der Lysosomen bewirkt, dass sich die Krebszellen aufgrund der Freisetzung der Enzyme selbst verdauen. Dies führt zu einem selektiveren und fokussierteren Behandlungseffekt und ermöglicht es, niedrigere Dosen von Photosensitizern und auch niedrigere Dosen von Licht zu verwenden.

Man unterscheidet hier zwischen Zylinder-Diffusoren mit typ. aktiven Längen von 10 bis 50 mm, Spot-Diffusoren, die einen vorwärts gerichteten Beleuchtungskegel erzeugen sowie Punktstrahler, die eine radiale Lichtemission aufweisen. Bei den Zylinder-Diffusoren kommt es im Betriebszustand insbesondere auf eine möglichst homogene seitliche Abstrahlung der Diffusor-Elemente über deren Länge an. Dies sowohl axial, d.h. an allen Punkten entlang jeder Linie vom proximalen zum distalen Ende in Richtung der Längsachse ist die Abstrahlungsintensität im Rahmen der Homogenitätsanforderung gleich, als auch radial, d.h. an allen Punkten jeder Umfangslinie entlang der Längsachse ist die Abstrahlungsintensität im Rahmen der Homogenitätsanforderung ebenfalls gleich, womit diese Diffusoren nahezu als Lambert'sche Strahler wirken. Gleichzeitig soll bei vielen Anwendungen auch eine hohe Streueffizienz erzielt werden, um möglichst einen geringen Wärmeeintrag ins Gewebe sicher zu stellen. Es gibt allerdings auch Anwendungen, bei denen ein gewisser Wärmeeintrag gefordert sein kann.

Bei den als Punktstrahlern ausgebildeten Diffusoren kommt es insbesondere auf eine über den gesamten sphärischen Raum gleichmäßige Lichtverteilung an.

Bekannte Beispiele sind Diffusor-Elemente aus einem dünnen Silikon-Zylinder, in die Streupartikel in Form von Titanoxid-Nano-Partikel eingelagert sind. Die Schrift DE 10 129 029 A1 beschreibt eine flexible Vorrichtung zur thermischen Verödung von biologischem Gewebe mittels Laserstrahlung mit einem die Laserstrahlung führenden Lichtleiter, dessen distales Ende von einem für die Laserstrahlung transparenten Hüllschlauch umgeben ist, welcher das Faserende überragt und in seinem vor dem Faserende liegenden Volumen mit einer Silikonmatrix gefüllt ist, in die Streupartikel eingebettet sind, wobei in eine Kunststoffmatrix, vorzugsweise aus Silikon, nicht-streuende Partikel mit Durchmessern von wenigen Nanometern, vorzugsweise aus Siliziumdioxid, in einem Konzentrationsbereich von vorzugsweise 1-10 % eingemischt sind und das distale Ende des Hüllschlauches durch ein für die Laserstrahlung transparentes oder opakes Endstück dicht verschlossen ist.

Diese können allerdings nur sehr aufwendig kostenintensiv mit ausreichender Abstrahlhomogenität hergestellt werden. Konglomerate der Streupartikel ergeben oft Abstrahlspots, bei denen die Intensität dann deutlich über dem Durchschnitt liegt. Zudem können nur geringere Laser-Leistungen appliziert werden.

Diese Lichtleiter mit den Diffusor-Elementen werden in der Regel nur einmal verwendet und nach jeder Behandlung entsorgt. Daher besteht auch ein gewisser Kostendruck, was die Herstellkosten betrifft. Daher wird verstärkt auch über wiederverwendbare Lösungen nachgedacht. Derartige Lösungen müssen dann entsprechend den einschlägig bekannten Normen aufbereitbar, beispielsweise desinfizierbar und/oder sterilisierbar sein. Als Aufbereitungsverfahren sind hier insbesondere Reinigungs-/ Desinfektionsverfahren mit stark basischen Lösungen und die Sterilisation mittels Autoklavieren bei Temperatur bis zu 135 °C und typischen Dampfdrücken von etwa 3 bar zu nennen. Typischerweise geht man dann von einigen Zehn bis mehreren Hundert derartiger Aufbereitungszyklen aus. Dies erfordert hohe Ansprüche an die thermische-, chemische und auch hydrolytische Beständigkeit. Daher eignen sich dann insbesondere Lichtleiter- und Diffusor-Ansätze aus Glas- oder Quarzglasfasern.

Bei der EVLT führt der behandelnde Arzt über eine winzige Punktionsstelle einen Katheder in die betroffene Vene ein, der als Leitschiene für den Venenlaser dient. Durch gezielte seitliche Abstrahlung der Laserenergie mittels des Diffusors wird die Gefäßinnenwand anschließend stark erwärmt, wodurch die Vene kollabiert und verschlossen wird. Der krankhafte Rückfluss des venösen Blutes wird somit unterbunden. In der Folge verhärtet die Vene, bildet sich zurück und kann vom Körper abgebaut werden. Dabei werden derzeit in der Regel sogenannte Ring- oder Doppelring-Fire-Systeme als Abstrahlelement verwendet. Das Laserlicht wird in Form eines relativ scharf begrenzten Ring- oder Doppelringlichtes radial an das die Vene umschließende Gewebes abgegeben. Dabei wird der Lichtleiter mit dem Abstrahlelement zur gleichmäßigen Behandlung oft manuell mit möglichst konstanter Geschwindigkeit durch den zu behandelnden Venenabschnitt gezogen, was die Applikation erschwert, da bei Nichtbeachtung beziehungsweise zu langer Verweildauer an einer Stelle weitere Zellschädigungen entstehen können.

Vorteile würde hier ein Zylinder-Diffusor mit sich bringen, wie er bei PDT-Anwendungen zum Einsatz kommt. Allerdings sind bei der EVLT-Behandlung deutlich höhere Laser-Leistungen erforderlich. So beträgt die Laserleistung typisch zwischen 10 und 50 W bei Wellenlängen im NIR-Bereich, d.h. zwischen etwa 800 nm und 1.480 nm, welche derzeit mit Dioden-Lasern (zum Beispiel 810 nm, 940 nm oder 1.480 nm) oder Nd: YAG-Lasern (1.064 nm) bereitgestellt wird. Inzwischen haben sich auch größere Wellenlängen um 2 $\mu$m zur EVLT-Behandlung etabliert. Zum Einsatz kommen dann beispielsweise Tm:YAG-Laser (1,9 $\mu$m) und Ho:YAG-Laser (2,1 pm). Aufgrund der Absorptionseigenschaften von Gewebe werden bei diesen Wellenlängen geringere Laserleistungen, typischerweise < 10 W, benötigt. Allerdings kommen hier bereits zwingend Quarzglas-Lichtleiter insbesondere für die Zuführung des Laserlichtes zum Einsatz.

Bei der LITT handelt es sich um ein minimal-invasives Verfahren, das zur lokalen Tumordestruktion eingesetzt wird. Dabei wird unter bildgebender Kontrolle (zum Beispiel Sonographie / MRT) der Tumor punktiert, eine (oder mehrere) Laserfaser(n) in den Tumorherd eingebracht und dieser durch thermische Energie verödet. Zum Einsatz kommen hier insbesondere Nd:YAG-Lasern (1.064 nm) sowie Diffusor-Tip-Applikatoren. Die Laserleistung liegt bei etwa 5 bis 8 W (s. u. a. "Laserinduzierte Interstitielle Thermotherapie (LITT) bei malignen Tumoren", BÄK und KBV 01/2002) .

Weitere Diffusor-Ansätze sind aus folgenden Schriften bekannt, wobei diese in vier Kategorien eingeteilt werden können: Volumenstreuende Diffusoren, Fasern mit aufgebrachten Streupartikeln, Diffusoren, die mittels Laserbearbeitung hergestellt werden und Diffusoren, die aus seitlich emittierenden Fasern gebildet sind.

Volumenstreuende Diffusoren sind beispielsweise in der Schrift EP 3 184 885 A1 beschrieben. Diese beschreibt einen Diffusor am Ende einer Lichtleitfaser aus Quarzglas, wobei zur Erzeugung des Diffusors vorgesehen ist, auf dem distalen Faserende der Lichtleitfaser eine Streumasse aufzubringen und diese zu dem Diffusor zu verfestigen. Das Aufbringen der Streumasse umfasst die Schritte (a) Bereitstellen einer $SiO_2$-Körnung, die amorphe $SiO_2$-Teilchen enthält und die zu mindestens 90 Gew.-% aus $SiO_2$ besteht, (b) Bereitstellen eines Hohlköpers aus Glas mit einer Hohlraumwandung, die einen nach außen offenen Hohlraum umgibt, (c) Bilden einer Schüttung der $SiO_2$-Körnung in dem Hohlraum und Einbringen des Faserendes in den Hohlraum, so dass mindestens ein Teil des Faserendes in die Schüttung hineinragt, (d) thermisches Verdichten der Schüttung unter Ausbildung einer porenhaltigen und zu mindestens 90 Gew.-% aus $SiO_2$ bestehenden Sintermasse, die mindestens teilweise von einer Glashülle umgeben ist. Nachteilig bei derartigen Ansätzen ist, dass diese volumenstreuenden Ansätze einen stark exponentiellen Abfall der Intensität mit sich bringen. Auch sind poröse Materialien mit Blick auf deren Aufbereitbarkeit in medizintechnischen Anwendungen nicht bevorzugt. Zudem können während der Behandlung Körperflüssigkeiten in das streuende, poröse Material eindringen, so dass die Streuwirkung stark verändert oder gar aufgehoben wird.

In der Schrift US 6,810,184 B2 wird ein Ansatz beschrieben, bei dem nanoporöse Siliziumdioxid-plattierte optische Fasern verwendet werden, um Fasern mit integral gebildeten Diffusionsspitzen herzustellen, die mit anderen Fasern verschmolzen werden können. Die offenbarten Diffusoren können zylindrisch hergestellt werden, wobei das Licht entlang seiner Länge diffundiert, sphärisch mit Licht, das nach außen in einem sphärischen Muster ausstrahlt, oder benutzerdefinierte, um unregelmäßige Flächen oder Volumina zu beleuchten. Gradienten- und Stufenindexeigenschaften können ebenfalls erreicht werden.

Die Schriften EP 2 062 077 A4, US 2009/0204111 A1 und DE 10 2015 119 875 A1 beschreiben Diffusoren, bei den zur ihrer Erzeugung mittels Laser in beziehungsweise auf der Faser Strukturen ein- beziehungsweise aufgebracht werden.

Die Schrift EP 2 062 077 A4 beziehungsweise WO 2008/024397 A2 beschreibt u.a. einen Diffusor zum Ausgeben von optischer Energie hoher Leistungsdichte zu einer Behandlungsstelle am distalen Ende mindestens einer optischen Faser, wobei der Diffusor ein Abschnitt einer vorbestimmten Länge des distalen Endes mindestens einer optischen Faser ist, sowie Streuungszentren, die in dem Abschnitt der vorbestimmten Länge am distalen Ende der optischen Faser positioniert sind, wobei die Streuungszentren bewirken, dass ein Teil der eingegebenen optischen Energie radial auf eine Behandlungsstelle austritt. Dabei ist vorgesehen, dass die Streuungszentren in der vorbestimmten Länge des Faserkerns oder in oder nahe einer Grenzfläche zwischen dem Faserkern und der Umhüllung in der vorbestimmten Länge angeordnet sind. Die Streuungszentren sind Defekte des Faserkerns, wie beispielsweise Nanocracks oder Nano-Hohlräume, die lokalisierte Brechungsindex-Differentiale entweder in dem Kern oder in oder nahe der Grenzfläche zwischen dem Kern und der Umhüllung erzeugen. Die Streuungszentren können Streupartikel sein, die in dem Kern oder der Umhüllung des Kerns enthalten sind. Neben aufwendiger und schwer kontrollierbarer Einbringung bzgl. beispielsweise der Verteilung und/oder Größe der oben genannten Nanocracks oder Nano-Hohlräume, können diese sich auch negativ auf die Bruchanfälligkeit des Bauteiles auswirken. Des Weiteren ist bei allen Ansätzen entweder damit zu rechnen, dass, bei ausreichend homogener Ausgestaltung der Streuzentren, aufgrund des exponentiellen Abfalls der seitlichen Emission oder ungleichmäßiger Verteilungen eine seitliche Abstrahlung nicht mit geforderter Homogenität erzielt wird.

Die Schrift US 2009/0204111 A1 beschreibt ein Laser Delivery System mit einer optischen Faser mit (i) einem Kern und einer Mantelschicht, die mindestens einen Teil des Kerns bedeckt, wobei die Mantelschicht einen niedrigeren Brechungsindex als der Kern aufweist, und (ii) einen Nicht-Merkmalsabschnitt und einen Merkmalsabschnitt mit Merkmalen, die das Licht dazu zwingen, sich radial von dem Merkmalabschnitt auszutragen und ein gewünschtes radiales Lichtausgangsmuster zu schaffen. Dabei ist vorgesehen, dass die Merkmale aus der Gruppe ausgewählt sind, die aus spiralförmigen Strukturen, radialen Schnitten, axialen Schnitten und einer Kombination davon besteht.

Die Schrift DE 10 2015 119 875 A1 beschreibt einen Lichtwellenleiter umfassend einen lichtwellenführenden Kern, einen Bereich im Lichtwellenleiter, wobei in dem Bereich des Lichtwellenleiters Mikromodifikationen angeordnet sind und wobei die Anordnung der Mikromodifikationen geordnet ist. Das Verfahren zur Einbringung der Mikromodifikationen in Lichtwellenleitern umfasst die Schritte (a) Fixierung eines Lichtwellenleiters in einer Halterung, wobei der Lichtwellenleiter und/ oder die Halterung beweglich gelagert sind, (b) Fokussieren von hochenergetischer Strahlung in eine Fokuslage, wobei die Fokuslage im Inneren des Lichtwellenleiters positionierbar ist, wobei die Strahlung von einer Strahlungsquelle im Pulsbetrieb erzeugt wird und wobei die Fokussiereinrichtung zum Fokussieren der hochenergetischen Strahlung beweglich gelagert ist und (c) Bewegen der Fokuslage durch den Lichtwellenleiter, wobei die Bewegung der Fokuslage im Inneren des Lichtwellenleiters in Abhängigkeit der Repetitionsrate gewählt wird.

Die Schrift WO 2017/103796 A1 beschreibt ein weiteres Beleuchtungssystem zur Abgabe von Licht. Das Beleuchtungssystem beschreibt dabei einen Lichtleiter, welche mit einem lichtstreuenden Element verbunden ist.

Eine weitere Schrift der Anmelderin PCT/EP2018/076487 beschreibt ein Beleuchtungssystem insbesondere für ein medizintechnisches Therapie- und/oder Diagnosesystem, umfassend wenigstens eine Laser-Lichtquelle und einen Lichtleiter, der an einem proximalen Ende an die wenigstens eine Laser-Lichtquelle anschließbar und/oder dieser zuordenbar ist, und welches am distalen Ende des Lichtleiters ein zylinderförmiges Diffusor-Element mit einer Längsachse aufweist, die senkrecht zu einer Einkoppelfläche des Lichtleiters in das oder in dem Diffusor-Element verläuft, wobei das Diffusor-Element im Betriebszustand Licht über seine aktive Länge seitlich zur Längsachse abstrahlt. Das Diffusor-Element weist dabei mindestens einen Diffusor-Grundkörper auf, der mindestens ein Streuelement beinhaltet, wobei vorzugsweise das zumindest eine Streuelement entlang der Längsachse des Diffusor-Grundkörpers verläuft. Mit einer derartigen Anordnung kann im Betriebszustand eine homogene Intensitätsverteilung der seitlichen Abstrahlung entlang der Länge des Diffusors erreicht werden. Dies erfordert aber auch eine größere Ausdehnung des Diffusor-Grundkörpers in Längsrichtung.

Aufgabe der Erfindung ist es daher, insbesondere für ein Beleuchtungssystem, insbesondere für ein medizintechnisches Therapie- und/oder Diagnosesystem, einen Diffusor zur Verfügung zu stellen, welcher noch kompakter ist als ein länglicher Diffusor, und welcher insbesondere als quasi Punktstrahler homogen, idealerweise sphärisch in den Raum bzw. in das umliegende Gewebe abstrahlt.

Vorteilhaft wäre es daher, ein kompaktes Diffusor-Element bereitzustellen, bei dem die Nachteile der eingangs beschriebenen Ansätze, wie z.B. Porosität und geringe Laser-Belastbarkeit, deutlich reduziert sind. Vorteilhaft wäre es dabei, wenn dieses Diffusor-Element dabei gleichmäßig mit hoher Effizienz abstrahlen kann. Es ist weiterhin Aufgabe der Erfindung, ein geeignetes Verfahren zu dessen kostengünstigen Herstellung bereitzustellen.

Offenbarung der Erfindung:

Die Aufgabe der Erfindung wird durch den Gegenstand der unabhängigen Ansprüche gelöst, wobei vorteilhafte Weiterentwicklungen den jeweiligen abhängigen Ansprüchen sowie der weiteren Offenbarung der Beschreibung und der Zeichnungen zu entnehmen sind.

Gegenstand der Erfindung ist demnach in einem ersten Aspekt ein Beleuchtungssystem, insbesondere für ein medizintechnisches Therapie- und/oder Diagnosesystem, umfassend wenigstens eine Laser-Lichtquelle und einen Lichtleiter, der an einem proximalen Ende an die wenigstens eine Laser-Lichtquelle anschließbar und/oder dieser zuordenbar ist, und welches am distalen Ende des Lichtleiters ein Diffusor-Element aufweist, welches im Wesentlichen eine radiale, sphärische Abstrahlcharakteristik aufweist, wobei das Diffusor-Element mindestens einen Diffusor-Grundkörper umfasst und der Diffusor-Grundkörper ein anorganisches Material, insbesondere ein Glas, eine Glaskeramik, einen glasähnlichen Werkstoff oder einen Verbundwerkstoff aus den zuvor genannten Werkstoffen und zumindest ein Streuelement umfasst, wobei die Oberfläche des zumindest einen Diffusor-Grundkörpers einer feuerpolierten Oberflächenqualität entspricht und wobei die Lichteinkopplung über eine Einkoppelfläche erfolgt, welche durch eine Verbindungszone des Diffusor-Grundkörpers ausgebildet ist.

Diese feuerpolierte Oberflächenqualität des Diffusor-Grundkörpers kann durch das Material und die Bearbeitung des Diffusor-Grundkörpers selbst erzeugt werden, wenn beispielsweise als Material ein Glas oder eine Glaskeramik ausgewählt wird. Eine feuerpolierte oder äquivalente Oberflächenqualität zumindest bzgl. des Parameters Porenfreiheit des Diffusor-Grundkörpers kann aber auch über eine Hülle bewirkt werden, wobei der zumindest eine Diffusor-Grundkörper von Vorteil zumindest teil- oder abschnittsweise eine Hülle umfasst, die diesen zumindest teil- oder abschnittsweise oder vollständig umschließt und welche die porenfreie und glatte Oberfläche bildet.

Eine porenfreie und glatte Oberfläche Diffusors ist damit im Wesentlichen auch dicht, was den Vorteil einer verringerten Reaktion mit Flüssigkeiten bzw. eine geringere Beeinflussung des Abstrahlverhaltens durch einwirkende Flüssigkeiten führt.

Als ein Streuelement wird nachfolgend ein Element bezeichnet, welches eine Streuung bewirken kann, vorzugsweise im Betrieb. Hierzu gehört ein Streubereich, der einen Volumenbereich umfassen kann und die Streuung bewirkt. Die Streuung selbst wird durch zumindest ein Streuzentrum im Streubereich bewirkt. Ein Streuzentrum kann beispielsweise ein Partikel oder eine Pore umfassen. Dabei ist bei der Herstellung des erfindungsgemäßen Beleuchtungssystems unter anderem vorgesehen, mittels einer Wärmebehandlung eine Dichte und eine Größenverteilung dieser Streuzentren definiert einzustellen und somit die Streuwirkung gezielt zu beeinflussen.

Das Beleuchtungssystem kann eine Laser-Lichtquelle umfassen, welche im Betriebszustand Licht, also elektromagnetische Strahlung, in einem bestimmten Spektralbereich aussendet. In einer besonders bevorzugten Ausführungsform der Erfindung für medizinische Therapien, etwa für die sogenannte photodynamische Therapie (PDT-Anwendungen), werden vorzugsweise Laser verwendet, die auf den zuvor verabreichten biochemisch modifizierten Farbstoff (Photosensitizer) abgestimmte Wellenlänge üblicherweise im sichtbaren Bereich, beispielsweise im grünen Spektralbereich bei 532 nm oder im roten Spektralbereich bei zum Beispiel 690 nm, aussenden.

Der Lichtleiter ist vorzugsweise mit einem Stecker an seinem proximalen Ende an die Laser-Lichtquelle angeschlossen. Als proximales Ende wird hierbei das Ende des Lichtleiters bezeichnet, in welches Licht eingekoppelt wird. Am distalen Ende weist der Lichtleiter das Diffusor-Element auf. Dabei sind Lichtleiter und Diffusor-Element in einer besonders bevorzugten Ausführungsform derart ausgebildet, dass sie direkt oder mittels einer Kanüle in ein Tumor-Gewebe eingebracht werden kann, welches sich innerhalb eines gesunden Gewebes gebildet hat. Als distales Ende wird hierbei das andere Ende des Lichtleiters bezeichnet, welches in der Regel zu dem proximalen Ende des Lichtleiters entfernt angeordnet ist und aus welchem insbesondere Licht austritt.

Die Laserstrahlung kann dabei über eine Lichteinkopplung am Diffusor-Element in das Diffusor-Element gelangen und wird in diesem mehrfach gestreut und über die Oberfläche des Diffusor-Elements im Wesentlichen radial sphärisch abgestrahlt

Eine radiale, sphärische Abstrahlcharakteristik des Diffusor-Elements meint im Sinne der vorliegenden Erfindung eine kugelförmige Abstrahlung, welche im Idealfall gleichmäßig ist. Eine in diesem Sinne perfekt kugelförmige Abstrahlcharakteristik entspricht demnach einer Abstrahlung, welche ausgehend von einem Punkt winkelunabhängig gleichmäßig ausgebildet ist und winkelunabhängig eine gleiche Intensität aufweist, vergleichbar einem Lambert'schen Strahler.

Dabei kann eine gewisse Winkelabhängigkeit der Abstrahlung zwar gegeben oder für bestimmte Ausführungsformen sogar gewünscht sein, was bedeutet, dass unter bestimmten Winkeln die Abstrahlung stärker oder schwächer sein kann, also eine unterschiedliche Abstrahlungsintensität in Bezug auf den Winkel vorliegt. Die Winkelabhängigkeit der Abstrahlung kann auch gezielt genutzt werden, um eine Inhomogenität in der Abstrahlung, etwa in Folge unterschiedlicher Konzentrationen der Streuzentren oder aufgrund unterschiedlicher Weglänge durch den Diffusor-Grundkörper, zu kompensieren.

Bevorzugt kommt es aber auf eine möglichst homogene Abstrahlung in eine das Diffusor-Element umgebende Kugelsphäre an. Insbesondere sind Intensitätsspitzen zu vermeiden. Durch eine photoinduzierte biochemische Reaktion, wie sie eingangs beschrieben ist, kann es nach einer Behandlung im Zuge einer photodynamischen Therapie idealerweise zu einem Absterben des Tumor-Gewebes kommen.

In der Regel können als Lichtleiter Quarz-Fasern verwendet werden, wobei die Stecker in der Regel als koaxialer Steckverbinder, sogenannte SMA-Stecker oder auch FC-Stecker, ausgebildet sein können, bei denen Fasern in den Stecker geklebt sind. Vorteilhaft hinsichtlich der thermischen Belastbarkeit können auch Stecker mit Neusilber-Hülsen sein, bei denen der Lichtleiter in die Neusilber-Hülse formschlüssig durch plastische Verformung eingebracht oder gecrimpt ist.

Darüber hinaus können bei größeren Laser-Leistungen auch Stecker zum Einsatz kommen, bei denen das Faserende des Lichtleiters durch ein Kegelprisma geschützt ist, was vorteilhaft bei Fehljustagen sein kann.

Der Lichtleiter kann bei den meisten Ausführungsformen Quarzglas mit einem Kern mit einem Brechungsindex $n_1$ und einem Kerndurchmesser DC von üblicherweise zwischen 50 und 1.000 $\mu$m, bevorzugt zwischen 200 und 600 $\mu$m, ausgestattet sein, sowie einen Mantel mit einem Brechungsindex $n_2$ umfassen, wobei gilt $n_1 > n_2$. Üblicherweise hat eine derartige Faser noch eine äußere als Buffer bezeichnete Polymerschicht, z.B. bestehend aus Polyamid oder Polyimid. Die damit üblicherweise erzielbare numerische Apertur NA beträgt etwa 0,22. Bekannt sind auch Quarzglas-basierte Lichtleiter, die mittels bestimmter Dotierstoffe auch eine numerische Apertur NA von bis zu 0,4 aufweisen. Die Lichteinkopplung erfolgt über eine Einkoppelfläche, welche durch eine Verbindungszone des Diffusor-Grundkörpers ausgebildet ist.

Zum Erzielen der gewünschten Abstrahlcharakteristik weist der Diffusor-Grundkörper vorzugsweise bestimmte geometrische Dimensionen und Verhältnisse auf, auf die nachfolgend eingegangen werden soll.

Hierzu weist der Diffusor-Grundkörper vorzugsweise eine im Wesentlichen kugelförmige, elliptische, tropfenförmige und/oder zylindrische Geometrie auf, um der Forderung nach einer im Wesentlichen radialen, sphärischen Abstrahlcharakteristik zu entsprechen. Gleichzeitig ermöglichen derartige kugelförmige, elliptische oder tropfenförmige Geometrien auch höchst vorteilhaft eine sehr kompakte Bauform des Diffusor-Grundkörpers. Mit von der Erfindung umfasst sind dabei auch Kombinationen dieser Grundformen, also Diffusor-Grundkörper, welche eine aus diesen Grundformen zusammengesetzte Geometrie aufweisen. Weitere Grundformen umfassen beispielsweise auch einen kurzen Zylinderabschnitt oder z.B. tropfenförmige oder ovale Geometrien oder auch kuppenförmige Geometrien. Die Erfinder haben herausgefunden, dass die gewünschte Abstrahlcharakteristik auch mit einer zylindrischen oder länglichen Geometrie des Diffusor-Grundkörpers erreicht werden kann, wenn ganz bestimmte Randbedingungen für derartige Bauformen in Abhängigkeit von dem Material und den Streuzentren eingehalten werden, auf die weiter unten vertiefend eingegangen wird.

Dabei ist ferner nicht ausgeschlossen, dass der Diffusor-Grundkörper einen Bereich der Oberfläche umfasst, welcher geometrisch von diesen Grundformen abweicht. Dieses ist dem Umstand geschuldet, dass der Diffusor-Grundkörper mit dem Lichtleiter verbunden ist, was am einfachsten über einen entsprechend abgeflachten Bereich der Oberfläche des Diffusor-Grundkörpers realisiert werden kann. Dieser abgeflachte Bereich kann demnach eben oder plan ausgebildet sein, wobei aber davon ausgegangen wird, dass die verbleibende Oberfläche des Diffusor-Grundkörpers eine hiervon abweichende Geometrie aufweist und insbesondere nicht plan bzw. eben ausgebildet ist.

Die radiale, sphärische Abstrahlcharakteristik wird weiterhin begünstigt durch eine Ausdehnung des Diffusor-Grundkörpers, wobei die größte Ausdehnung LD des Diffusor-Grundkörpers in einer ersten Richtung nicht größer ist als das 10-fache, bevorzugt das 5-fache und besonders bevorzugt das 2,5-fache der Ausdehnung des Diffusor-Grundkörpers in einer zweiten Richtung DD senkrecht zu dieser ersten Richtung, weiterhin bevorzugt nicht größer als das 2-fache und nochmals besonders bevorzugt nicht größer als das 1,5-fache. Die Ausdehnung in der Richtung DD meint hierbei eine Ausdehnung in einer Richtung senkrecht zur Richtung der Lichteinkoppelung, also senkrecht zur Längsrichtung des Lichtleiters im Bereich der Einkoppelung. Die Ausdehnung in der Richtung LD meint die Länge des Diffusor-Grundkörpers entlang seiner Längsachse, sofern dieser z.B. eher oval oder elliptisch ausgeprägt ist, also parallel zur Richtung der Lichteinkoppelung bzw. in einer Richtung parallel zur Längsrichtung des Lichtleiters im Bereich der Einkoppelung. Als grundsätzlich vorteilhaft hat sich herausgestellt, wenn gilt:

$$LD \geq DD.$$

In einer besonders bevorzugten Ausführungsform ist die Ausdehnung des Diffusor-Grundkörpers in einer ersten Richtung gleich der Ausdehnung des Diffusor-Grundkörpers in einer zweiten Richtung senkrecht zu dieser ersten Richtung, wodurch eine kugelförmige Ausbildung gegeben ist.

Die maximale Ausdehnung des Diffusor-Grundkörpers LD in einer Richtung kann in einem Bereich zwischen 200 $\mu$m und 10 mm oder sogar darüber liegen. Eine günstige Länge und/oder Breite des Diffusor-Grundkörpers hängt dabei von mehreren Faktoren ab. So beeinflusst die Art der Streuzentren und deren Konzentration im Volumen des Diffusor-Grundkörpers die Streulänge LS, womit die Entfernung von der Licht-Einkoppelstelle bezeichnet wird, bei der die Lichtintensität auf den Wert 1/e bzw. auf 36,8 % abgefallen ist. Hiernach richtet sich günstigerweise die maximale Ausdehnung LD des Diffusor-Grundkörpers, wobei es sich als vorteilhaft für eine homogene Abstrahlcharakteristik herausgestellt hat, wenn die maximale Länge LD nicht größer ist als das Dreifache der Streulänge, so dass gilt:

$$LD \leq 3 * LS,$$

bevorzugt LD ≤ 2,5 * LS und

besonders bevorzugt LD ≤ 2 * LS.

In einer besonders bevorzugten Ausführungsform ist LD = LS.

Bei einer Streulänge von 10 mm kann der Diffusor-Grundkörper dann von Vorteil ebenfalls 10 mm lang sein. Günstige Ausführungsformen liegen allgemein zwischen 250 μm und 4 mm, und bevorzugt zwischen 300 μm und 3 mm.

Die Breite des Diffusor-Grundkörpers richtet sich von Vorteil nach dem Kerndurchmesser des Lichtleiters. Der Lichtleiter kann eine Einzelfaser, beispielsweise Mono-oder Multimodelichtleitfaser umfassend einen Kern mit einem Kerndurchmesser und einen Mantel oder ein Faserbündel mit einem Faserbündeldurchmesser umfassen. Der äußeren Durchmesser des Lichtleiters liegt üblicherweise für die zum Einsatz kommenden Quarzfasern zwischen 200 μm und 800 μm.

In einer bevorzugten Ausführungsform kann der Lichtleiter eine Einzelfaser mit einem Kern mit einem Kerndurchmesser und einem Mantel umfassen, wobei der Durchmesser des Diffusor-Grundkörpers im Bereich der Einkoppelfläche größer oder zumindest gleich groß ist wie der Kerndurchmesser des Lichtleiters im Bereich der Einkoppelfläche. Ebenfalls bevorzugt beträgt dabei das Verhältnis von Kerndurchmesser des Lichtleiters und Durchmesser oder Breite des Diffusor-Grundkörpers ≤ 1,0 bis 0,7, besonders bevorzugt von ≤ 1,0 bis 0,8.

In einer weiteren bevorzugten Ausführungsform kann der Lichtleiter ein Faserbündel umfassen mit einem Faserbündeldurchmesser, wobei der Durchmesser des Diffusor-Grundkörpers im Bereich der Einkoppelfläche größer ist als oder gleich groß wie der Faserbündeldurchmesser des Lichtleiters im Bereich der Einkoppelfläche. Ebenfalls bevorzugt beträgt dabei das Verhältnis von Faserbündeldurchmesser des Lichtleiters und Durchmesser oder Breite des Diffusor-Grundkörpers ≤ 1,0 bis 0,7, besonders bevorzugt von ≤ 1,0 bis 0,8.

In einer weiteren Ausführungsform kann der Lichtleiter als starrer Faserstab ausgebildet sein. Hierbei kann vorgesehen sein, dass der Diffusor-Grundkörper auf das distale Ende des starren Glasfaserstabs aufgeklebt oder aufgespleißt ist. Dazu kann vorgesehen sein, dass der Glasfaserstab am distalen Ende zu einem Konus derart verjüngt ist, dass der Durchmesser der distalen Endfläche nahezu gleich groß ist wie der Durchmesser des Diffusor-Grundkörpers. Weiterhin kann vorgesehen sein, dass der starre Faserstab mindestens eine Biegung aufweist. Auf diese Weise ist es besonders gut möglich, das erfindungsgemäße Beleuchtungssystem mit einem starren Faserstab und einem Diffusor-Element im DentalBereich einzusetzen, etwa zur Behandlung von Mukositis. Dabei ist es vorteilhaft, dass zu den im Dentalbereich eingesetzten sogenannten Curing-Rods ein kompatibles System bereitgestellt werden kann. Derartige Curing-Rods, z.B. bekannt aus der DE 10 2013 208 838 A1, bestehen i.d.R. aus Faserstäben, die gebogen und verjüngt ausgeführt sein können, mit denen Curing-Rods beispielsweise Zahnfüllungen mit blauem Licht ausgehärtet werden können.

Für die meisten Anwendungen der erfindungsgemäßen Beleuchtung ist es günstig, wenn die Dimensionen des Diffusor-Grundköpers derart ausgewählt werden, dass das Beleuchtungssystem im Betriebszustand eine Abstrahlung mit einer geringen prozentualen Abweichung von einer mittleren Abstrahlungsintensität ermöglicht. Dabei ist es für die meisten vorgesehenen Anwendungen besonders günstig, wenn die Intensitätsverteilung der Abstrahlung um höchstens ± 30 % und bevorzugt höchstens als ± 20 % von einem gleitenden Durchschnittswert abweicht, um einen optimalen Behandlungserfolg zu erzielen. Eine geringere Abweichung, etwa von höchstens 15 % oder sogar höchstens 10 %, ist für die meisten Anwendungen noch besser. Ausgenommen hiervon sind Anwendungsfälle, bei denen eine bestimmte Abweichung in der Abstrahlcharakteristik gewünscht ist.

Zur Bestimmung der Abstrahlungsintensität kann die Intensität, Leuchtdichte, Luminanz oder Helligkeit mehrerer Punkte, etwa entlang einer Geraden auf der Oberfläche des Diffusor-Grundkörpers, gemessen werden. Als mittlerer Abstrahlungsintensität kann dann der Mittelwert aller auf der Geraden gemessen Werte zugrunde gelegt werden.

Die Angabe, dass das Beleuchtungssystem im Betriebszustand eine Intensitätsverteilung der Abstrahlung aufweist, die um höchstens ± 30 % und bevorzugt höchstens als ± 20 % von einem Durchschnittswert, insbesondere von einem gleitenden Durchschnittswert abweicht, legt für diese Abweichung einen gleitenden Durchschnitt zugrunde, wobei die Mittelung über eine Mehrzahl von auf der Geraden nebeneinanderliegender Punkte verstanden wird.

Am Übergangsbereich zwischen Lichtleiter und Diffusor-Grundkörper sind bevorzugt Einrichtungen und/oder Maßnahmen zur Homogenisierung der seitlichen Abstrahlung, also in einer Richtung senkrecht zur Längsrichtung des Lichtleiters, vorgesehen. Beispielsweise gehören hierzu Schichten am distalen Ende des Lichtleiters, um eine vorwärts gerichtete Abstrahlung aus dem distalen Ende zu verhindern beziehungsweise diese zurück zu reflektieren und damit den Streuprozessen im Diffusor-Grundkörper erneut bereitzustellen und andererseits Streulichteffekte und/oder Lichtreflexe am Diffusor-Grundkörper zu vermeiden.

Damit lassen sich reproduzierbar und auch kostenoptimiert, im Betriebszustand homogen abstrahlende Diffusor-Elemente für medizinische Therapien, wie sie eingangs erwähnt sind, bereitstellen.

Unterstützt werden kann die Homogenisierung der Intensität der Abstrahlung, wenn der Durchmesser des Diffusor-Grundkörpers, in den die Streuelemente eingebettet sind, gleich groß oder größer ist als ein Kerndurchmesser oder Faserbündeldurchmesser des Lichtleiters. Der Durchmesser des Diffusor-Grundkörpers betrifft dabei die Ausdehnung des Diffusor-Grundkörpers in einer Richtung senkrecht zur Längsrichtung des Lichtleiters und entspricht damit dem oben genannten Wert DD.

Als besonders günstig hat sich ein Verhältnis zwischen Kerndurchmesser bzw. Faserbündeldurchmesser des Lichtleiters und Durchmesser des Diffusor-Grundkörpers von ≤ 1,0 bis 0,3, besonders bevorzugt von ≤ 1,0 bis 0,5 herausgestellt.

Ein nur geringfügig gegenüber dem Durchmesser kleinerer Kerndurchmesser bzw. Faserbündeldurchmesser kann dabei einen Intensitäts-Peak an der Einkoppelstelle, also dem Übergangsbereich von Lichtleiter und Diffusor-Grundkörper, reduzieren.

Ein deutlich kleinerer Kerndurchmesser bzw. Faserbündeldurchmesser gegenüber dem Durchmesser des Diffusor-Grundkörpers, das heißt ein Verhältnis von < 0,8, kann dagegen zu einer Intensitätsabsenkung an der Einkoppelstelle führen, die für bestimmte Anforderungen ebenfalls von Vorteil sein kann.

Liegt das Verhältnis zwischen 1 und 0,9, hat sich zudem herausgestellt, dass eine besonders robuste mechanische Kopplung bzw. Verbindung, zum Beispiel mittels Spleißen, zwischen Lichtleiter und Diffusor-Grundkörper erzielt werden kann.

Andererseits sind auch Ausführungsbeispiele denkbar, bei den beispielsweise eine als nahezu Kugel ausgeführter Diffusor-Grundkörper mit 1 mm Durchmesser an eine Faser mit einem Kerndurchmesser von 400 $\mu$m verbunden, insbesondere angespleißt, ist. Hier kann das Verhältnis auch deutlich kleiner als 1 sein, in diesem Beispiel beträgt das Verhältnis 0,4.

In bevorzugter Ausführungsform weist das Diffusor-Element zwischen dem Diffusor-Grundkörper und dem distalen Ende des Lichtleiters eine Verbindungszone auf, die form- und/oder stoffschlüssig mittels Verkleben, Spleißen oder Verpressen hergestellt ist und die den Diffusor-Grundkörper und den Kerndurchmesser oder den Faserbündeldurchmesser des Lichtleiters verbindet. Der Begriff Spleißen meint in diesem Zusammenhang ein festes, stoffschlüssiges Verbinden von Diffusor-Grundkörper und Lichtleiter durch ein Anschmelzen, also ein Erweichen durch Temperaturbeaufschlagung, zumindest eines der beiden zu verbindenden Körper, vorzugsweise des Lichtleiters, und anschließendem Inkontaktbringen. Eine feste Verbindung kann sich dann bei einem Abkühlen einstellen.

Hierzu kann ein brechwertangepasster, hochtransparenter Kleber verwendet werden. Beim Spleißen wird mittels einer Corona-Entladung und/oder mittels eines Lasers, üblicherweise mit einem $CO_2$-Laser, der Lichtleiter und der Diffusor-Grundkörper an- beziehungsweise aufgeschmolzen und zusammengeführt. Je nach Material, welches für den Diffusor-Grundkörper und dem Lichtleiter verwendet wird, kann es erforderlich sein, dass zur Anpassung der Wärmeausdehnungskoeffizienten ein Zwischenmedium verwendet wird. Dieses kann beispielsweise bei einer Glas-/ Quarz-Verschmelzung ein Lot- oder Übergangsglas oder ein optischer Kleber oder Kitt sein.

Zum Anpassen gegebenenfalls unterschiedlicher thermischer Ausdehnungskoeffizienten kann es von Vorteil sein, wenn zwischen dem Diffusor-Grundkörper und dem distalen Ende des Lichtleiters zusätzlich in der Verbindungszone ein Zwischenmedium vorgesehen ist. Dieses kann beispielsweise ein Übergangsglas oder Lotglas sein. Andererseits kann dies auch ein transparenter dauerelastischer Kleber sein. Des Weiteren kann in der Verbindungszone ein optisches Element angeordnet sein oder die Verbindungszone als optisches Element ausgeführt sein, um beispielsweise die Strahlführung und/oder Lichtlenkung durch geometrische oder Anpassung von Brechwerten zu modifizieren.

Zum Schutz des Diffusor-Grundkörpers ist ferner von Vorteil eine Hülle vorgesehen, welche in einer bevorzugten Ausführungsform den Diffusor-Grundkörper vorzugsweise zumindest teil- oder abschnittsweise oder auch vollständig umschließt, wobei es vorteilhaft sein kann, wenn zusätzlich mindestens auch die Verbindungszone zwischen Diffusor-Grundkörper und Lichtleiter umschlossen ist. Damit kann die mechanische Belastbarkeit der Verbindung Lichtleiter/Diffusor-Grundkörper erhöht werden. Von Vorteil weist der Lichtleiter in diesem Bereich keine Buffer-Schicht auf, so dass diese Zone mit einer derartigen Hülle geschützt werden kann, ohne dass ein sogenannter Recoating-Prozess zum mechanischen Schutz erfolgen müsste.

In einer besonders vorteilhaften Ausführungsform weist der Diffusor-Grundkörper keine ungeschützten Oberflächen mehr auf, weder in Richtung oder im Bereich der Einkoppelstelle noch in entgegengesetzter Richtung, welche nicht von der schützenden Hülle bedeckt sind. Dies bietet einen zusätzlichen und festen Schutz der Oberfläche des Diffusor-Grundkörper und/oder der Einkoppelstelle, etwa vor äußeren Beschädigungen der Oberfläche des Diffusor-Grundkörpers wie Rissen oder Kratzer infolge der Verwendung, was zu einer veränderten Abstrahlcharakteristik führen könnte und was damit sehr nachteilig für die vorgesehenen Anwendungen sein kann. Dadurch kann die Langlebigkeit des Beleuchtungssystems gesteigert werden.

In einer weiteren besonders vorteilhaften Ausführungsform umschließt die Hülle zumindest die Verbindungszone zwischen Diffusor-Grundkörper und Lichtleiter und sorgt auf diese Weise für eine besonders hohe Stabilität der Verbindung, da der Außenbereich der Verbindung vor äußeren Einflüssen besonders gut geschützt ist. Eine Hülle, welche zumindest die Verbindungszone zwischen dem Diffusor-Grundkörper und dem Lichtleiter schützt und umschließt bietet einen weiteren großen Vorteil. Da der Lichtleiter hier über keine Buffer-Schicht verfügt, ist dieser Bereich anfällig für Korrosion, etwa in Verbindung mit Wasserdampf in der Atmosphäre oder durch Kontakt mit Flüssigkeiten oder Feuchtigkeit während der Verwendung. Eine feuchtigkeitsdichte Hülle gerade in diesem Bereich schützt demnach auch den Lichtleiter im Kontaktbereich mit dem Diffusor-Grundkörper sicher vor Korrosion und verlängert damit ebenso die Langlebigkeit des Beleuchtungssystems.

Die Hülle kann dabei eine für seitlich aus dem Diffusor-Element emittiertes Licht transparente oder transluzente Schicht, bestehend aus Flüssig-Silikon, thermoplastischem Polymer, Schmelzkleber, 2-komponentigen Kleber oder Sol-Gel-Glas, aus einem Schrumpfschlauch oder aus zusätzlich aufgesetzten transparenten oder transluzenten Aufsatzelementen umfassen, die den Diffusor-Grundkörper und/oder die Übergangsstelle zwischen Diffusor-Grundkörper und Lichtleiter umschließen. Als vorteilhaft hat sich auch eine Lackschicht herausgestellt, welche zusätzliche lichtstreuende Pigmente, z.B. in Form von Titanoxid, Aluminiumoxid oder Kalziumcarbonat, enthält. Damit kann zusätzlich eine Homogenisierung der Abstrahlung erreicht werden. Die Schicht kann beispielsweise als Tauchschicht oder durch Bestreichen der Oberfläche aufgebracht sein.

Denkbar ist auch ein Einschluss mit einem vergleichsweise niedrig schmelzenden Glas, welches eine niedrige Verarbeitungstemperatur von vorzugsweise kleiner 500 °C, bevorzugt kleiner als 400 °C und besonders bevorzugt kleiner als 300 °C aufweist. Bei der Auswahl eines geeigneten Glases ist darauf zu achten, dass die Erweichungs- bzw. Verarbeitungstemperatur des ausgewählten Glases niedriger ist als diejenige des Diffusor-Elements oder des Diffusor-Grundkörpers. Hierdurch wird gewährleistet, dass es bei dem Aufbringen des niedrig schmelzenden Glases auf den Diffusor-Grundkörper keine unerwünschten Veränderungen, etwa bezüglich der Streuzentren und damit der Abstrahlcharakteristik, auftreten können. Von Vorteil ist die Verarbeitungstemperatur des aufzubringenden Materials um wenigstens 50 K, bevorzugt wenigstens 100 K niedriger als die Erweichungstemperatur des Diffusor-Elements. Hierbei ist zu berücksichtigen, dass das Diffusor-Element verschiedene Materialien umfassen kann, etwa auch den Kern und/oder Mantel des Lichtleiters, sowie den Diffusor-Grundkörper. Von Vorteil werden daher diese ggf. unterschiedlichen Erweichungstemperaturen mitberücksichtigt.

Die Hülle weist dabei eine bestimmte minimale und eine maximale Dicke auf, so dass einerseits ein hinreichender Schutz des Diffusor-Grundkörpers gegeben ist und andererseits das Diffusor-Element insgesamt nicht zu groß für die geplanten Verwendungen wird. Eine zu dick ausgebildete Hülle kann auch zu einer unerwünschten Schwächung der Abstrahlung führen.

Typische Schichtdicken einer Lack-Schicht liegen bei etwa 10 $\mu$m bis 100 $\mu$m, wobei auch mehrlagige Schichten vorgesehen sein können. Typische Dicken eines Schlauches beginnen bei etwa 5 $\mu$m und gehen bis etwa 500 $\mu$m.

Vorzugsweise umfasst das Diffusor-Element zumindest ein Streuelement bzw. ist als Streuelement ausgebildet und umfasst zumindest ein Streuzentrum. Die radiale, sphärische Abstrahlcharakteristik wird bei der vorliegenden Erfindung durch Streuung des in den Diffusor-Grundkörper eingeleiteten Lichts in dem Streubereich bewirkt, in welchem die Streuung stattfindet.

Verantwortlich für die Streuung sind die Streuzentren, welche in den Streubereich eingelagert sind. Im Sinne der Erfindung sind Streuzentren grundsätzlich alle Partikel und/oder Materialagglomerationen und/oder inhomogene Bereiche, gleich welcher Form, welchen Materials und/oder welcher Größe, die das Licht streuen können. Die Streuzentren können durch klassische Streuung, insbesondere Rayleigh- und/oder Mie-Streuung, ebenso wie durch Beugung und/oder Reflektion sowie Mehrfachprozessen dieser Mechanismen untereinander ihre streuende Wirkung entfalten. Ihre Funktion ist lediglich, individuell oder in ihrer Summe auftreffendes Licht abzulenken.

Ebenso denkbar sind optisch aktive Pigmente, beispielsweise Leuchtstoffe, also Stoffe, die eine Lumineszenz infolge einer Anregung zeigen können. Diese können beispielsweise bestimmte Phosphore umfassen, die eingestrahltes Licht einer bestimmten Wellenlänge in ein Licht einer anderen Wellenlänge konvertieren und abstrahlen können. Neben diesen durch ihre Phosphoreszenz gekennzeichneten Stoffen können beispielsweise auch Stoffe, die über eine Fluoreszenz verfügen, verwendet werden. Derartige Materialien sind auch als organische oder anorganische Phosphore, die sich in eine Matrix aus inaktivem Material, beispielsweise Kunststoffe (Epoxy und Silikon), Glas, Glaskeramik oder glasartige Werkstoffe oder Keramik einbetten lassen, oder als keramische Konverter, sog. Optokeramiken, den Phosphor bilden, bekannt. Damit lassen sich auch Abstrahlungen mit überlagerten Spektren aus der eingestrahlten und abgestrahlten Wellenlänge realisieren.

Eine Mehrzahl von Streuzentren kann in einer bestimmten vorgebbaren geometrischen Anordnung im Volumen, vorzugsweise um den Mittelpunkt des Diffusor-Grundkörpers herum, angeordnet sein.

Denkbar sind unterschiedliche Anordnungen und/oder Konzentrationen der Streuzentren im Volumen des Diffusor-Grundkörpers.

So kann in einer bevorzugten Ausführungsform vorgesehen sein, die Streuzentren in einer regelmäßigen Struktur und homogenen Konzentration in dem Volumen des Diffusor-Grundkörpers anzuordnen, vorzugsweise um den Mittelpunkt herum, was zu einer gleichmäßigen Konzentration im Volumen führt. Die Dichte der Streuzentren kann durch chemische oder thermische Prozesse eingestellt werden, so dass eine homogene, aber auch eine inhomogene Verteilung über das Gesamtvolumen einstellbar ist.

In einer weiteren Ausführungsform kann auch ein Verbundkörper als Streuelement vorgesehen sein, welcher zumindest ein stark streuendes Material umfasst, beispielsweise ein hochdotiertes Weißglas. Die Streuung kann durch gezielt eingelagerte Streuzentren lokal eingestellt werden.

In einer anderen Ausführungsform kann auch vorgesehen sein, dass eine Kernzone um den Mittelpunkt des Diffusor-Grundkörpers herum keine oder eine deutlich reduzierte Anzahl von Streuzentren je Volumeneinheit gegenüber der Anzahl von Streuzentren je Volumeneinheit außerhalb der Kernzone aufweist und somit die Streuzentren überwiegend außerhalb dieser Kernzone angeordnet sind. Die Kernzone betrifft dabei vorzugsweise ein den Mittelpunkt umgebendes Volumen mit einer Ausdehnung, welche nicht größer ist als die Hälfte der größten Ausdehnung des Diffusor-Grundkörpers in einer Richtung. Auf diese Weise kann erreicht werden, dass das eingekoppelte Licht, welches in der Regel mit geringer NA (< 0,3, typischerweise um die 0,2) eingekoppelt wird, nicht sofort an den Streuzentren gestreut wird, sondern homogen mit gleicher Intensität kugelförmig abgestrahlt wird.

Bei dieser Ausführungsform unterscheidet sich demnach die Streuzentrendichte im oberflächennahen Bereich des Diffusor-Grundkörpers von derjenigen im mittelpunktsnahen Bereich des Diffusor-Grundkörpers, bevorzugt ist dabei die Streuzentrendichte im oberflächennahen Bereich grösser als im mittelpunktsnahen Bereich; besonders bevorzugt liegt ein Gradient der Streuzentrendichte vor. Der Begriff Streuzentrendichte meint hierbei die Dichte, also die Anzahl an Streuzentren pro Volumeneinheit.

Zu den Streuzentren gehören u.a. Poren, Partikel, Kristallite, Polykristallite, poröse und/oder pigmentierte und/oder optisch aktive Pigmente, z.B. in Form von Phosphore und/oder eingefärbte Bereiche, beispielsweise eingefärbte Partikel, eingefärbte Kristallite oder eingefärbte Pigmente, oder Färbungen des Glases, oder Inhomogenitäten in Form von Brechungsindexschwankungen oder beliebige Kombinationen derartiger Streuzentren, wobei die Inhomogenitäten des anorganisches Materials Phasenseparation, Entmischungen und/oder partikuläre Einlagerung, Keime und/oder Kristallite umfassen.

Dabei können vorteilhaft auch Kombinationen der vorstehend beispielhaft genannten Streuzentren in dem anorganischen Material vorliegen. Die Inhomogenitäten eines Glases oder einer Glaskeramik, welche die Streuelemente bei Glas- oder Glaskeramikmatrixlösungen bilden können, umfassen beispielsweise Phasenseparationen, Entmischungen und/oder partikuläre Einlagerungen, Keime und/oder Kristallite.

Im Fall von Glas oder Glaskeramik als anorganisches Material für den Diffusor-Grundkörper können als Streuzentren bevorzugt Streupartikel in das Glas oder die Glaskeramik eingelagert werden, oder aber die Streuzentren werden durch inhomogene Bereiche des Glases oder der Glaskeramik gebildet, in das sie eingelagert sind.

Derartige Streuzentren auf der Basis von Streupartikeln im Glas oder in einer Glaskeramik oder inhomogene Bereiche des Glases oder der Glaskeramik sowie die zugrundeliegenden Verfahren zur Herstellung werden beispielsweise in der Schrift WO 2009/100834 der Anmelderin beschrieben.

Im Fall des Einsatzes von Streupartikeln als Streuzentren werden bevorzugt Streupartikel verwendet, deren Schmelztemperatur größer ist als die Schmelztemperatur des Glases oder der Glaskeramik, in welche sie eingebettet sind. Weil die Streupartikel in diesem Fall zumindest ihre streuenden Eigenschaften beim Herstellungsprozess nicht verändern, wird ihre Auswahl erleichtert und sie können entsprechend als Rohmaterial zugekauft werden.

Bevorzugt weisen die Streupartikel einen Durchmesser zwischen 10 nm und 5.000 nm auf, besonders bevorzugt zwischen 100 nm und 1.200 nm. Für nicht runde Streupartikel wird als Durchmesser im Sinne der Erfindung ihre maximale Ausdehnung verstanden. Dies gilt auch für Größenangaben an andere Stellen, wo ebenfalls die Angabe eines Durchmessers als maximale Ausdehnung in einer Richtung verstanden werden soll, sofern das zugrundeliegende Objekt keinen Durchmesser aufweist. Streupartikel mit den oben genannten Dimensionen sind beispielsweise gut geeignet in Verbindung mit einem Weißglas als Material für den Diffusor-Grundkörper.

Diese Abmessung können auch für andere Formen der Streuzentren gelten, etwa für Poren, Kristallite oder Inhomogenitäten, wobei durch Phasentrennung oder Entmischung entstandene Inhomogenitäten eher kleinere Durchmesser, beispielsweise in einem Bereich von 10 nm bis 1.000 nm oder von 100 nm bis 800 nm aufweisen können. So werden als vorteilhafte Größen von Partikeln oder Poren in einem Borosilikatglas etwa 200 nm bis 700 nm oder 200 nm bis 500 nm angenommen.

Die Streupartikel können aus einer Vielzahl von Materialen ausgewählt sein. Bevorzugt bestehen sie im Wesentlichen aus $SiO_2$ und/oder $BaO$ und/oder $MgO$ und/oder $BN$ und/oder $AIN$ und/oder $SN$ und/oder $ZrO_2$ und/oder $Y_2O_3$ und/oder $AI_2O_3$ und/oder $TiO_2$ und/oder $Ru$ und/oder $Os$ und/oder $Rh$ und/oder $Ir$ und/oder $Ag$ und/oder $Au$ und/oder $Pd$ und/oder $Pt$ und/oder diamantartiger Kohlenstoff und/oder Glaskeramik-Partikel. Mischungen von Streupartikeln aus verschiedenen Materialien, Verbindungen und/oder Konglomerate aus diesen oder auch miteinander verschmolzene und/oder gesinterte Streupartikel sind ebenfalls denkbar ebenso wie die metallischen Komponenten der vorgenannten Oxide und Nitride alleine.

Die Effizienz der Auskopplung aus dem Streubereich ist neben der streuenden Eigenschaft der Streupartikel als intrinsischem Parameter auch von der Konzentration der Streupartikel im Streubereich selbst abhängig. Über die Wahl der Konzentration der Streuzentren im Streubereich ist eine Skalierung der Emission möglich.

Eine beispielhafte Ausführungsform sieht daher vor, dass die Streuzentren durch Streupartikel gebildet werden, wobei die Konzentration der Streupartikel im Streubereich von 10 ppm bis 1.000 ppm und bevorzugt von 20 ppm bis 100 ppm beträgt. Hierbei bezieht sich die Konzentrationsangabe in ppm auf den Anteil der Streupartikel im Verhältnis zu den Masseanteilen der Bestandteile des jeweiligen anorganischen Materials, in welchem die Streupartikel eingelagert sind. Auch im Fall von andersartigen Streuzentren, beispielsweise Poren, Inhomogenitäten im Material oder Kristallite im Fall einer Glaskeramik, haben sich diese Konzentrationen als hilfreich herausgestellt.

Dienen beispielsweise inhomogene Bereiche des Glases oder der Glaskeramik als Streuzentren, ergibt sich eine alternative Ausführungsform der Erfindung, in der die inhomogenen Bereiche bevorzugt durch Phasentrennung und/oder Entmischung der Komponenten des Glases oder der Glaskeramik gebildet werden, in welches sie eingelagert sind.

Der Diffusor-Grundkörper selbst umfasst gemäß der Erfindung, wie zuvor beschrieben, ein anorganisches Material, insbesondere ein Glas, eine Glaskeramik, ein Quarzglas oder einen glasähnlichen Werkstoff oder einen Verbundwerkstoff aus den zuvor genannten Materialien. Besonders bevorzugt werden aber Gläser, Mehrkomponentengläser oder Glaskeramik oder Verbundwerkstoffe aus diesen Materialien verwendet, da sie ein einfacheres und besseres Einstellen der Abstrahlcharakteristik ermöglichen.

Glas oder Glaskeramik als Material für den Diffusor-Grundkörper ist besonders geeignet, da es, beispielsweise im Vergleich zu Kunststoffen, wesentlich robuster und vor allem thermisch stabiler ist, so dass auch größere Laserleistungen applizierbar sind.

Für die vorgesehenen Verwendungen kann ein Material für den Diffusor-Grundkörper als besonders geeignet angesehen werden, welches sich neutral gegenüber elektromagnetischer Strahlung in demjenigen Wellenlängenbereich verhält, welcher für die vorgesehene Verwendung ausgewählt ist und demzufolge vorzugsweise von der Lichtquelle abgestrahlt wird. Der für Erfindung relevante Wellenlängenbereich elektromagnetischer Strahlung liegt bei etwa 0,4 μm bis etwa 2,2 μm. Für Phototherapie-Anwendungen sind typische Bereiche im sichtbaren Spektrum, insbesondere im roten Spektralbereich zwischen 600 nm und 700 nm, insbesondere bei 690 nm, oder im NIR-Bereich zwischen 700 nm und 1000 nm. EVLT-Anwendungen zielen hier eher auf Wellenlängen von 800 nm bis 2,2 μm ab, typischerweise in einem Bereich von 980 nm bis 1.100 nm, um die 1.500 nm und Bereiche zwischen 1,9 μm bis 2,2 μm.

Erfindungsgemäß ist in einer ersten Ausführungsform vorgesehen, für den Diffusor-Grundkörper ein Glas mit oder ohne Eigenfärbung auszuwählen, bei welchem die gewünschte Wellenlänge der elektromagnetischen Strahlung nicht absorbiert wird. Dies kann ein Weißglas sein, welches weiße Pigmente umfasst, um einen weißlichen Farbeindruck zu bewirken.

In einer besonders bevorzugten Ausführungsform umfasst der Diffusor-Grundkörper ein Silikat-Weißglas. Dieses weist eine extreme Streuwirkung auf. Vorzugsweise handelt es sich dabei um ein As-Pb-haltiges Silikatglas. Ein solches Glas ist ein Silikatglas, welches Blei (Pb) und Arsen (As) beinhaltet. Im Fall von inhomogenen Bereichen als Streuelement können diese inhomogenen Bereiche einen gegenüber dem umgebenden Glas erhöhten Gehalt an Blei und/oder Arsen aufweisen. Alternativ können selbstverständlich auch Streuelemente, etwa Streupartikel, eingelagert sein und die Streuzentren ausbilden.

Ein besonders geeignetes Glas für den Diffusor-Grundkörper ist ein Na-Al-K-As-Pb-Silikatglas. Dieses kann wenigstens 25 Gew.-% Bleioxid im Glas, bevorzugt wenigstens 30 Gew.-% Bleioxid umfassen. Inhomogene Bereiche, welche die Streuelemente darstellen, beispielsweise tröpfchenförmige Entmischungszonen mit erhöhtem Bleigehalt, bilden dann Bleiarsenat mit beispielsweise 38 Gew.-% Blei oder darüber hinaus aus, wobei diese Entmischungszonen einen Durchmesser zwischen 100 nm und 600 nm aufweisen können. Das Na-Al-K-As-Pb-Silikatglas kann 3 Gew.-% Arsenoxid oder mehr umfassen. Eine mögliche Zusammensetzung eines erfindungsgemäß geeigneten Na-Al-K-As-Pb-Silikatglases zeigt Tabelle 1.

Tabelle 1: Zusammensetzung eines erfindungsgemäß geeigneten Na-Al-K-As-Pb-Silikatglases

|  | Ma.-% | Ma.-% |  | mol/100 g | mol/100 g |  | mol-% | mol-% |
| --- | --- | --- | --- | --- | --- | --- | --- | --- |
|  | inhomogen | homogen | M | inhomogen | homogen |  | inhomogen | homogen |
| SiO2 | 47,1 | 47,6 | 60,084 | 0,7839 | 0,7922 |  | 69,09 | 69,77 |
| Al2O3 | 1,7 | 1,7 | 101,961 | 0,0167 | 0,0167 |  | 1,47 | 1,47 |
| PbO | 34,4 | 34,4 | 223,199 | 0,1541 | 0,1541 |  | 13,58 | 13,57 |
| Na2O | 3,6 | 3,4 | 61,979 | 0,0581 | 0,0549 |  | 5,12 | 4,83 |
| K2O | 6 | 5,6 | 94,195 | 0,0637 | 0,0595 |  | 5,61 | 5,24 |
| CaO | 1,42 | 1,4 | 56,079 | 0,0253 | 0,0250 |  | 2,23 | 2,20 |

(fortgesetzt)

|  | Ma.-% | Ma.-% |  | mol/100 g | mol/100 g |  | mol-% | mol-% |
|---|---|---|---|---|---|---|---|---|
| As2O3 | 4,9 | 5 | 197,841 | 0,0248 | 0,0253 |  | 2,18 | 2,23 |
| Fe2O3 | 0,03 | 0,03 | 159,691 | 0,0002 | 0,0002 |  | 0,02 | 0,02 |
| P2O5 | 1 | 1 | 141,943 | 0,0070 | 0,0070 |  | 0,62 | 0,62 |
| ZnO | 0,07 | 0,05 | 81,379 | 0,0009 | 0,0006 |  | 0,08 | 0,05 |
|  |  |  |  |  |  |  |  |  |
| Sum | 100,22 | 100,18 |  | 1,1347 | 1,1354 |  | 100,00 | 100,00 |

In einer besonders bevorzugten Ausführungsform wird ein Na-Al-K-As-Pb-Silikatglas als Farbglas mit weißer Anmutung mit 30 Gew.-% Bleioxid und 3 Gew.-% Arsenoxid ausgewählt. Es werden keine Streupartikel eingebracht, sondern bei der Erzeugung des zu Beginn transparenten und farblosen Farbglases entmischt dieses, so dass Entmischungszonen mit einer Größe von 50 nm bis 500 nm Durchmesser entstehen, welche gleichmäßig verteilt sind. Diese Entmischungszonen sind Bleiarsenat und verfügen über einen wesentlich größeren Brechungsindex als das Grundglas, wodurch sich der Streueffekt ergibt. Dieses nun weiße, opake Farbglas kann dann zu den gewünschten Geometrien für den Diffusor-Grundkörper weiterverarbeitet werden, also beispielsweise zu einer kugelförmigen Geometrie.

Selbstverständlich sind auch andere Gläser, auch bleifreie bzw. schwermetallfreie oder -arme Gläser, möglich und geeignet für die Erfindung.

Beispiele für solche Gläser für den Diffusor-Grundkörper aus dem Bereich der bleifreien Zinn-Silikat-Gläser beziehungsweise Alkali-Zink-Silikat Gläser beinhalten folgende Komponenten (angegeben in Gew.-% auf Oxidbasis):

Tabelle 2: Zusammensetzungsbereich eines bleifreien Zinn-Silikat-Glases in Gew.-%

|  | von | bis |
|---|---|---|
| $B_2O_3$ | 0 | 24 |
| $SiO_2$ | 23 | 62, 1 |
| $Al_2O_3$ | 0 | 10 |
| $Li_2O$ | 0 | 10 |
| $Na_2O$ | 0 | 18,5 |
| $K_2O$ | 0 | 25,7 |
| BaO | 0 | 57, 8 |
| ZnO | 0 | 40 |
| $La_2O_3$ | 0 | 25 |
| $ZrO_2$ | 0 | 10 |
| $HfO_2$ | 0 | 14,2 |
| $SnO_2$ | >0 | 2 |
| MgO | 0 | 8 |
| CaO | 0 | 8 |
| SrO | 0 | 24,4 |
| $Ta_2O_5$ | 0 | 22 |
| $Y_2O_3$ | 0 | 11,9 |
| $Rb_2O$ | 0 | 15 |

(fortgesetzt)

|  | von | bis |
|---|---|---|
| $Cs_2O$ | 0 | 21 |
| $GeO_2$ | 0 | 7,5 |
| F | 0 | 2 |
| $\Sigma\ R_2O$ | 5 | 20 |
| $\Sigma$ MgO, CaO, SrO, ZnO | 20 | 42 |

So kann beispielsweise ein Na-Al-K-Ca-Zn-Silikatglas verwendet werden, welches blei- und arsenfrei ist, was unter Umweltgesichtspunkten von Vorteil sein kann. Auch hier ist es möglich, beispielsweise kugelförmigen Poren im Volumen auszubilden, welche in diesem Fall kalziumreiche Ablagerungen darstellen. Durch diese Inhomogenitäten im Glas werden die Streuzentren ausgebildet. Die erreichbare Größe der Poren liegt bei bis zu 500 nm. Die kalziumreichen Ablagerungen in diesen Entmischungszonen bestehen aus $CaF_2$. Weitere geeignete Gläser sind die Gläser vom Typ N-BK7, optisches Bor-Kronglas der Anmelderin oder Borosilikatglas. In diesem Zusammenhang ist beispielsweise ein weiteres für die Erfindung sehr geeignetes Material zu nennen, welches geeignet ist, Mie-Streuung zu induzieren. Die Mie-Streuung meint eine Streuung, welche entsteht, wenn ein Fehler, etwa eine Inhomogenität, in ihrer Größe mit der aktuellen Wellenlänge vergleichbar ist, d. h. größer als 10 % der Wellenlänge ist.

Die Herstellung und verschiedene Konfigurationen derartige Materialien sind in der Druckschrift WO 2014/165048 A1 bzw. DE 11 2014 001 293 T5 der Anmelderin beschrieben. Erfindungsgemäß kann der Diffusor-Grundkörper aus porösem oder phasengetrenntem Glas hergestellt sein, welches ausgebildet ist, eine Streuung in der gewünschten Abstrahlrichtung zu optimieren, während zur selben Zeit der gewünschte Raumwinkel von gestreutem Licht in der gewünschten Abstrahlrichtung erreicht wird.

Deshalb wird gemäß einer weiteren Ausführungsform der Erfindung ein streuender Diffusor-Grundkörper bereitgestellt, welcher aus einem Glassystem ausgebildet ist, das eine Phasentrennung durchläuft, wie z.B. das klassische Borosilikatglassystem; die Systeme $K_2O\text{-}SiO_2$, $K_2O\text{-}Li_2O\text{-}SiO_2$, $K_2O\text{-}Na_2O\text{-}SiO_2$ und $K_2O\text{-}BaO\text{-}SiO_2$ (Kawamoto and Tomozawa, 1981, J. Amer. Ceram. Soc., vol. 64 (5), 289-292); das System $MgO\text{-}Al_2O_3\text{-}SiO_2$ (Zdaniewski, 1978, J. Amer. Ceram. Soc., vol. 61 (5-6), Seiten 199-204); und das System $CdF_2\text{-}LiF\text{-}AlF_3\text{-}PbF_2$ (Randall et al., 1988, J. Amer. Ceram. Soc., vol. 71 (12), Seiten 1134-1141)].

Diese können mittels einer kontrollierten thermischen Behandlung einer Phasentrennung unterworfen werden, wobei sich die Glaszusammensetzung in zwei Phasen trennt, und wahlweise einem weiteren Eingriff zum Erzeugen einer offenen Porosität innerhalb des Glases durch einen Laugeschritt, um ein Glas mit Poren in der Größenordnung von 200 bis 700 nm, z. B. 200 bis 500 nm, bevorzugt von 300 bis 500 nm, besonders bevorzugt von 300 bis 450 nm, mit einer Anzahldichte von annähernd $10^8$ bis $10^{12}$ mm$^{-3}$, bevorzugterweise $10^9$ bis $10^{11}$ mm$^{-3}$, insbesondere bevorzugterweise $10^{10}$ bis $10^{11}$ mm$^{-3}$, zu erhalten.

Gemäß einem weiteren Aspekt der Erfindung wird ein Diffusor-Grundkörper mit einem Streuelement bereitgestellt, der durch Schmelzen eines Borosilikatglases, bevorzugt eines Alkali-Borosilikatglases, hergestellt ist, das, nachdem es zur Entspannung langsam gekühlt wurde, einer Phasentrennung unter Verwendung einer gut kontrollierten thermischen Behandlung unterworfen wird, wobei die Zusammensetzung sich in eine silikatreiche Phase und eine borreiche Phase trennt, und wahlweise einem weiteren Eingriff zur Erzeugung offener Porosität innerhalb des Glases durch einen Säurelaugungsschritt und einen Poren-reinigenden kaustischen Laugungsschritt ausgesetzt, um ein Glas mit Poren in der Größenordnung von 200 bis 700 nm, z. B. 200 bis 500 nm, bevorzugt von 300 bis 500 nm, besonders bevorzugt von 300 bis 450 nm, mit einer Anzahldichte von annähernd $10^8$ bis $10^{12}$ mm$^{-3}$, bevorzugterweise $10^9$ bis $10^{11}$ mm$^{-3}$, insbesondere bevorzugterweise $10^{10}$ bis $10^{11}$ mm$^{-3}$ zu erhalten.

Gemäß eines weiteren Aspekts der Erfindung wird ein Diffusor-Element bereitgestellt, umfassend wenigstens eine optische Faser und wenigstens einen Mie-streuenden Diffusor-Grundkörper zum Zerstreuen von Licht, das von der wenigstens einen optischen Faser emittiert wird, wobei der wenigstens eine Mie-streuende Diffusor-Grundkörper ein phasengetrenntes oder poröses Glas (wie beispielsweise ein Borosilikatglas, bevorzugterweise ein Alkali-Borosilikatglas) mit disperse Phase-Partikeln mit einer Partikelgröße von 200 bis 700 Nanometer, z.B. 200 bis 500 Nanometer oder Poren mit einer Größe von 200 bis 700 Nanometer, z.B. 200 bis 500 Nanometer, bei einer Anzahldichte von $10^8$ bis $10^{12}$ mm$^{-3}$ umfasst.

Gemäß eines weiteren Aspekts der Erfindung wird ein Beleuchtungssystem bereitgestellt, umfassend eine optische Faser oder ein optisches Faserbündel und ein optisches streuendes Element, das an dem distalen Ende der optischen Faser oder des optischen Faserbündels angefügt ist, zum Zerstreuen von Licht, das von dem distalen Ende der optischen Faser oder des optischen Faserbündels emittiert wird, wobei das optische streuende Element ein phasengetrenntes oder poröses Glas (wie beispielsweise ein Borosilikatglas, bevorzugterweise ein Alkali-Borosilikatglas) mit disperse Phase-Partikeln mit einer Partikelgröße von 200 bis 700 Nanometer, z.B. 200 bis 500 Nanometer, oder Poren mit einer Größe von 200 bis 700 Nanometer, z.B. 200 bis 500 Nanometer, bei einer Anzahldichte von $10^8$ bis $10^{12}$ mm$^{-3}$, umfasst.

Die Bezugnahmen auf eine Partikelgröße von, zum Beispiel, 200 bis 700 Nanometer und eine Porengröße von, zum Beispiel, 200 bis 700 Nanometer sollen bedeuten, dass die relevanten Poren oder Partikel einen tatsächlichen Durchmesser von 200 bis 700 Nanometer aufweisen. So wird, zum Beispiel, in einem Diffusor-Grundkörper gemäß der Erfindung die Menge, d.h. die Anzahldichte, von Partikeln oder Poren mit einem tatsächlichen Durchmesser von z.B. 200 bis 700 Nanometer, $10^8$ bis $10^{12}$ mm$^{-3}$, (bevorzugterweise $10^9$ bis $10^{11}$ mm$^{-3}$, insbesondere bevorzugterweise $10^{10}$ bis $10^{11}$ mm$^{-3}$) betragen.

Der Diffusor-Grundkörper kann Partikel oder Poren mit einem tatsächlichen Durchmesser außerhalb des Bereiches von 200 bis 700 Nanometer umfassen, jedoch wird die Anzahl von Partikeln oder Poren mit einem tatsächlichen Durchmesser von 200 bis 700 Nanometer $10^8$ bis $10^{12}$ mm$^{-3}$ (bevorzugterweise $10^9$ bis $10^{11}$ mm$^{-3}$, insbesondere bevorzugterweise $10^{10}$ bis $10^{11}$ mm$^{-3}$) betragen.

Gemäß eines weiteren Aspekts der Erfindung wird ein Verfahren zur Herstellung eine Diffusor-Elements mit einem Mie-streuenden Diffusor-Grundkörper bereitgestellt, wobei das Verfahren umfasst: Herstellen eines Mie-streuenden optischen Elements durch Unterwerfen eines zur Entspannung langsam gekühlten Glases (wie beispielsweise ein Borosilikatglas, bevorzugterweise ein Alkali-Borosilikatglas) einer Phasentrennung unter Verwendung einer kontrollierten thermischen Behandlung, und wahlweise Unterwerfen des phasengetrennten Glases einer Säurelaugung, um Poren zu erzeugen, und einer kaustischen Laugung, um die resultierenden Poren zu reinigen, und Anfügen des resultierenden Mie-streuenden optischen Elements an das Ende einer optischen Faser oder an das Ende eines Bündels optischer Fasern.

Gemäß eines weiteren Aspekts wird ein Verfahren bereitgestellt zum Zerstreuen oder Streuen von Licht, das durch Transmittieren des Lichts durch ein optisches streuendes Element, vorzugsweise durch den Diffusor-Grundkörper, umfassend ein phasengetrenntes oder poröses Glas (zum Beispiel ein Borosilikatglas oder ein Alkali-Borosilikatglas) mit disperse Phase-Partikeln mit einer Partikelgröße von 200 bis 700 Nanometer, z.B. 200 bis 500 Nanometer oder Poren mit einer Größe von 200 bis 700 Nanometer, z.B. 200 bis 500 Nanometer, bei einer Anzahldichte von $10^8$ bis $10^{12}$ mm$^{-3}$, erfolgt.

Zum Beispiel kann das optische streuende Element durch Schmelzen eines Borosilikatglases, bevorzugterweise eines Alkali-Borosilikatglases, hergestellt werden, das nachdem es zur Entspannung langsam gekühlt wird einer Phasentrennung unter Verwendung einer gut kontrollierten thermischen Behandlung unterworfen wird, und wahlweise einen weiteren Eingriff zum Erzeugen offener Porosität innerhalb des Glases durch einen Säurelaugungsschritt und einen Poren-reinigenden kaustischen Laugungsschritt, um ein Glas zu erhalten, das Poren in der Größenordnung von 200 bis 700 Nanometer, z.B. 200 bis 500 Nanometer, bevorzugterweise 300-500 nm, insbesondere 300-450 nm, mit einer Anzahldichte von annähernd $10^8$ bis $10^{12}$ mm$^{-3}$, bevorzugterweise $10^9$ bis $10^{11}$ mm$^{-3}$, insbesondere bevorzugterweise $10^{10}$ bis $10^{11}$ mm$^{-3}$ aufweist.

Erfindungsgemäß induziert eine thermische Behandlung des Borosilikat-Mutterglases, bevorzugterweise ein Alkali-Borosilikatglas, vorzugsweise in einer der unten aufgeführten Zusammensetzungen, eine Glas-in-Glas-Phasentrennung. Die Phasen sind als eine silikatreiche Phase und eine Bor-reiche Phase definiert. Nachdem Durchlaufen der Wärmebehandlung, kann das Mutterglas dann chemisch gelaugt werden. Wenn das Mutterglas chemisch gelaugt wird, wird die Wirtsphase (engl. host phase) oder Massenphase (engl. bulk phase) als die silikatreiche Phase betrachtet und die Poren, die aus dem Entfernen der Borreichen Phase resultieren, werden als das streuende Merkmal betrachtet.

Um ein Glas zu erhalten, das eine derartige Kombination von Merkmalen im Nanobereich in der Größenordnung von 200 bis 700 nm, z.B. 200-500 nm, mit einer Anzahldichte von annähernd $10^8$ bis $10^{12}$ mm$^{-3}$ aufweist, sollte das Glas einem gut kontrollierten Wärmebehandlungsprofil unterworfen werden. Hinsichtlich kommerziell-verfügbarer Produkte ist zum Beispiel ein poröses Glas von der Fa. SCHOTT AG, Mainz, bekannt unter der Bezeichnung CoralPor™, etwa für Anwendungen in Chromatographie-Medien, Referenzelektrodenkreuzungen, als Wirtsmaterial für Sensoren und als ein Additiv (Füllstoff) in Beschichtungen (siehe James et al., US 2013/0017387). Während der Herstellung wird CoralPor™ poröses Glas einer vorsichtig kontrollierten Wärmebehandlung unterworfen, um eine Glasin-Glas Phasentrennung zu induzieren. Diese Wärmebehandlung bestimmt letztlich die endgültige Größe der in dem Material vorhandenen Streuungsmerkmale. Dies führt dazu, dass das Herstellungsverfahren manipuliert werden kann, um CoralPor™ poröses Glas in einer Form herzustellen, die den gewünschten Kriterien, d.h. 200 bis 700, z.B. 200-500 nm Merkmale bei einer Anzahldichte von $10^8$ bis $10^{12}$ mm$^{-3}$, genügt.

Erfindungsgemäß können die streuenden optischen Elemente hergestellt werden durch, zum Beispiel, Schmelzen eines Borosilikatglases, bevorzugterweise eines Alkali-Borosilikatglases, das, nachdem es zur Entspannung langsam gekühlt wurde, einer Phasentrennung mittels einer thermischen Behandlung mit kontrolliertem Temperatur-/Zeitverlauf in dem Temperaturbereich von 500 - 800 °C, wie beispielsweise 600 - 800 °C, bevorzugt 650-750 °C, bevorzugter 700 - 725 °C, für eine Zeitspanne von, zum Beispiel, 1 bis 150 Stunden, wie bspw. 24 bis 48 Stunden oder 48 bis 80 Stunden unterworfen wird. Zum Beispiel kann für eine gegebene Zusammensetzung die thermische Behandlung, die eingesetzt wird, um Merkmale im Nanobereich von etwa 200 Nanometer zu erreichen, eine Dauer von 20-26 Stunden bei 700 °C umfassen. Hier spielt auch die Abkühlphase eine Rolle, welche ebenfalls zu Erzielung einer optimalen bzw. erwünschten Streuwirkung hinsichtlich ihres Temperatur-/Zeitverlaufs entsprechend zu kontrollieren ist.

Die Bedingungen können, basierend auf den verwendeten Schmelzparametern (d.h. Temperatur und Abschreckverfahren (engl. quenching method)) und Zusammensetzung eingestellt werden. Allgemein ist das eingesetzte Verfahren abhängig von dem gewünschten Phasenwachstum. Zum Beispiel führt, innerhalb der Phasentrennungsregion, im Allgemeinen, eine erhöhte Temperatur für längere Dauer zu größeren Merkmalsgrößen, obwohl die Größe der Merkmale auch von der spezifischen Zusammensetzung abhängen wird.

Dieses thermische Behandlungsverfahren kann für einige Anwendungen als vollständig angesehen werden. Mit anderen Worten werden für einige Anwendungen keine weiteren Verfahrensschritte benötigt, um die gewünschten Ergebnisse von 200 nm bis 700 nm, z.B. 200-500 nm Merkmale mit einer Anzahldichte von annähernd $10^8$ bis $10^{12}$ $mm^{-3}$ zu erreichen. Das Basisglas kann zum Beispiel ein Borosilikatglas sein, das geeignete Siliziumdioxid- und Borat-Gehalte aufweist, damit eine Phasentrennung auftritt, und einen ausreichenden Boratgehalt aufweist, um die gewünschte Anzahldichte von Poren im Nanobereich zu erreichen. Bevorzugt umfasst das Borosilikatglas wenigstens einige Alkalimetalloxide. Zum Beispiel umfasst die Borosilikatglas-Zusammensetzung gemäß einer Ausführungsform der Erfindung (basierend auf Gew.-%):

| | |
|---|---|
| $B_2O_3$ | 15,00-40,00 |
| $SiO_2$ | 45,00-80,00 |
| $R_2O$ | 0,0-20,0 |
| R'O | 0,0-20,00 |
| $R''O_2$ | 0,0-10,00 |
| $Al_2O_3$ | 0,0-10,00 |

wobei $R_2O$ die Summe von $Li_2O$, $Na_2O$, $K_2O$, $Rb_2O$ und $Cs_2O$ ist (und bevorzugterweise größer als 0 ist),
R'O die Summe von BaO, CaO, MgO, SrO, PbO und ZnO ist, und $R''O_2$ die Summe von $TiO_2$, $ZrO_2$ und $HfO_2$ ist.

Eine weitere für die Erfindung geeignete Borosilikatglas-Zusammensetzung ist nachfolgend angegeben (basierend auf Gew.-%) :

65 % bis 85 % $SiO_2$
6 % bis 15 % $B_2O_3$
3 % bis 9 % Alkalioxide (Natriumoxid $Na_2O$; Kaliumoxid $K_2O$)
1 % bis 8 % $Al_2O_3$
0 % bis 5 % Erdalkalioxide (CaO, MgO, ...)

Mit Hinblick auf die in dieser Beschreibung genannten Bereiche umfassen alle Bereiche wenigstens die zwei Endpunkte der Bereiche, sowie alle Werte zwischen den beiden Endpunkten. Demnach soll, zum Beispiel, ein Bereich von 1 bis 10 so verstanden werden, dass er etwa die Werte 1,0, 1,5, 2,0, 2,8, 3,0, 3,1, 4,0, 4,2, 5,0, 5,3, 6,0, 7,0, 7,7, 8,0, 9,0, 9,9 oder 10,0 ausdrücklich offenbart.

In dem Borosilikatglas funktioniert $SiO_2$ als ein primärer Netzwerkbildner. Demnach umfasst gemäß einem weiteren Aspekt der Erfindung die Borosilikatglas-Zusammensetzung 45,00-80,00 Gew.-% an $SiO_2$, zum Beispiel 45,00-75,00 Gew.-% an $SiO_2$ oder 45,00-70,00 Gew.-% an $SiO_2$ oder 45,00-65,00 Gew.-% an $SiO_2$ oder 45,00-60,00 Gew.-% an $SiO_2$ oder 50,00-60,00 Gew.-% an $SiO_2$.

In dem Borosilikatglas funktioniert $B_2O_3$ als ein Netzwerkbildner und als primärer Bildner der Merkmale im Nanobereich des resultierenden phasengetrennten/porösen Glases. Demnach umfasst gemäß einem weiteren Aspekt der Erfindung die Borosilikatglas-Zusammensetzung 15,00-40,00 Gew.-% an $B_2O_3$, zum Beispiel 20,00-35,00 Gew.-% an $B_2O_3$ oder 20,00-30,00 Gew.-% an $B_2O_3$.

Gemäß einem weiteren Aspekt umfasst die Borosilikatglas-Zusammensetzung 0,00-20,00 Gew.-% an $R_2O$ (bevorzugt > 0,00-20,00 Gew.-% an $R_2O$), wobei $R_2O$ die Summe von $Li_2O$, $Na_2O$, $K_2O$, $Rb_2O$ und $Cs_2O$ ist, zum Beispiel 1,00-15,00 Gew.-% $R_2O$ oder 1,00-10,00 Gew.-% $R_2O$ oder 2,00-8,00 Gew.-% $R_2O$.

Gemäß einem weiteren Aspekt umfasst die Borosilikatglas-Zusammensetzung 0,00-20,00 Gew.-% an R'O (die Summe von BaO, CaO, MgO, SrO, PbO und ZnO), zum Beispiel 1,00-15,00 Gew.-% R'O oder 1,00-10,00 Gew.-% R'O oder 2,00-8,00 Gew.-% R'O. Die R'O-Metalloxide können zum Einstellen des Brechungsindex jeder Phase verwendet werden.

Gemäß einem weiteren Aspekt umfasst die Borosilikatglas-Zusammensetzung 0,00-10,00 Gew.-% an $R''O_2$ (die Summe von $TiO_2$, $ZrO_2$ und $HfO_2$), zum Beispiel 0,00-8,00 Gew.-% $R''O_2$ oder 1,00-8,00 Gew.-% $R''O_2$ oder 0,00-5,00 Gew.-% $R''O_2$ oder 1,00-5,00 Gew.-% $R''O_2$. Diese Metalloxide können verwendet werden, um die chemische Beständigkeit zu erhöhen und können zum Einstellen des Brechungsindex jeder Phase verwendet werden.

In der Borosilikatglas-Zusammensetzung agiert $Al_2O_3$ üblicherweise als ein Netzwerk-Co-Bildner und kann auch verwendet werden, um die chemische Beständigkeit zu erhöhen. Demnach umfasst gemäß einem weiteren Aspekt die Borosilikatglas-Zusammensetzung nach der Erfindung 0,00-10,00 Gew.-% an $Al_2O_3$, zum Beispiel 0,00-8,00 Gew.-% an $Al_2O_3$ oder 1,00-8,00 Gew.-% an $Al_2O_3$ oder 0,00-5,00 Gew.-% an $Al_2O_3$ oder 1,00-5,00 Gew.-% an $Al_2O_3$ oder 2,50-5,00 Gew.-% an $Al_2O_3$.

Gemäß einem weiteren Aspekt kann das Glas ein Glas gemäß der in James et al. (US 2013/0017387) beschriebenen

Glaszusammensetzung sein. Diese Glaszusammensetzung umfasst (basierend auf Gew.-%): 40-80 % $SiO_2$, 5-35 % $B_2O_3$ und 1-10% $Na_2O$, bevorzugt 45-65 % $SiO_2$, 20-30 % $B_2O_3$ und 2-8 % $Na_2O$ und besonders bevorzugt 50-55 % $SiO_2$, 25-27 % $B_2O_3$ und 5-7% $Na_2O$. Wie in der Schrift US 2013/0017387 offenbart, kann das Glas weitere Bestandteile, wie beispielsweise $ZrO_2$, $TiO_2$, $Al_2O_3$, $CaO$ und/oder $ZnO$ und wahlweise weitere Bestandteile, z. B. Oxide des Mg, Fe, Mn, Ce, Sn, etc., umfassen.

Die folgenden Tabellen 2-5 stellen weitere Beispiele geeigneter Basisgläser A bis T zur erfindungsgemäßen Verwendung dar.

Allgemein ist entscheidend, dass bei einer Entmischung Phasen mit höherem Brechungsindex neben Phasen mit niedrigerem Brechungsindex vorliegen, um eine Streuwirkung zu erzielen.

Tabelle 3: Glaszusammensetzungen auf Oxid-Gew.-%-Basis

| Oxide | A | B | C | D | E | F |
|---|---|---|---|---|---|---|
| $SiO_2$ | 52.40 | 52.77 | 50.93 | 56.85 | 54.85 | 56.85 |
| $B_2O_3$ | 25.99 | 25,87 | 25.73 | 30.31 | 30.31 | 30.31 |
| $Al_2O_3$ | 3.42 | 3.41 | 3.39 | | | |
| $Na_2O$ | 5.92 | 5.89 | 586 | 6.90 | 6.90 | 6.90 |
| $CaO$ | 5.14 | 5.12 | 5.09 | | | |
| $ZnO$ | | | | 3.00 | 4.00 | 2.00 |
| $TiO_2$ | 2.00 | | 2.00 | | | 2.00 |
| $ZrO_2$ | 5.14 | 6.94 | 7.00 | 3.00 | 4.00 | 2.00 |

Tabelle 4: Glaszusammensetzungen auf Oxid-Gew.-%-Basis

| Oxide | G | H | I | J | K | L |
|---|---|---|---|---|---|---|
| $SiO_2$ | 54.85 | 58.92 | 57.68 | 58.69 | 57.69 | 60.19 |
| $B_2O_3$ | 30.31 | 28.41 | 27.81 | 28.30 | 27.82 | 29.02 |
| $Al_2O_3$ | | | | | | |
| $Na_2O$ | 6.90 | 6.47 | 6.33 | 6.44 | 6.33 | 6.61 |
| $CaO$ | | | | | | |
| $ZnO$ | 3.00 | 3.10 | 4.09 | 2.06 | 3.05 | 2.10 |
| $TiO_2$ | 2.00 | | | 1.33 | 1.97 | |
| $ZrO_2$ | 3.00 | 3.09 | 4.09 | 3.18 | 3.14 | 2.80 |

Tabelle 5: Glaszusammensetzungen auf Oxid-Gew.-%-Basis

| Oxide | M | N | O | P | Q | R |
|---|---|---|---|---|---|---|
| $SiO_2$ | 56.86 | 57.47 | 53.28 | 50.61 | 52.70 | 52.12 |
| $B_2O_3$ | 27.43 | 27.72 | 26.43 | 26.23 | 26.14 | 25.86 |
| $Al_2O_3$ | | | | | | |
| $Na_2O$ | 6.24 | 6.31 | 6.02 | 5.97 | 5.95 | 5.89 |
| CaO | | | | | | |
| ZnO | 5.41 | 5.49 | 5.23 | 5.20 | 5.18 | 5.12 |
| $T.O_2$ | | | 2.00 | 6.98 | 2.00 | 2.01 |
| $ZrO_2$ | 4.03 | 3,01 | 7.03 | 5.00 | 8.02 | 8.99 |

Tabelle 6: Glaszusammensetzungen auf Oxid-Gew.-%-Basis

| Oxide | S | T |
|---|---|---|
| $SiO_2$ | 60.19 | 54.15 |
| $B_2O_3$ | 29.02 | 20.11 |
| $Al_2O_3$ | | 3.45 |
| $Na_2O$ | 6.61 | 5.68 |
| CaO | | |
| ZnO | 2.10 | 5.17 |
| $TiO_2$ | | |
| $ZrO_2$ | 2.08 | 5.18 |

Ein hinsichtlich seines Herstellprozesses besonders bevorzugter Diffusor-Grundkörper ergibt sich, wenn der Diffusor-Grundkörper aus Borosilikatglas, Zinnsilikatglas oder Alkali-Zink-Silikat-Glas und die Streuelemente aus Weißglas gebildet sind.

Gemäß einer nochmals weiteren Ausführungsform der Erfindung wird ein Diffusor-Grundkörper zur Verfügung gestellt, bei welchem der Diffusor-Grundkörper eine Glaskeramik umfasst oder aus einem glaskeramischen Material gefertigt ist. Die Verwendung eines glaskeramischen Materials für den Diffusor-Grundkörper bietet den Vorteil, dass der Diffusor-Grundkörper als Ganzes im Röntgenbild zumindest teilweise oder abschnittsweise erkennbar ist und somit die Position des Diffusors im Körper eines Patienten bestimmbar ist.

Glaskeramisches Material ist zudem extrem thermoschockbeständig und besitzt eine hohe spektrale Transmission bis zu einer Wellenlänge von etwa 2,5 $\mu$m, was es für die Erfindung besonders interessant macht. Dabei kann ein glaskeramisches Material nicht nur für den Diffusor-Grundkörper, sondern auch als Streuelement verwendet werden, beispielsweise eine Keatit-Glaskeramik, die durch einen geeigneten Temperprozess aus der Hochquarz-Mischkristall-Glaskeramik hergestellt werden kann. Weiterhin eignen sich auch Cordierit-Glaskeramiken oder Magnesium-Aluminium-Silikat Glaskeramiken als Diffusor-Grundkörper und/oder Streuelement.

Ein besonders geeignetes glaskeramisches Material für den Diffusor-Grundkörper und/oder die Streuzentren stellen die Glaskeramiken auf der Basis von Lithium-Alumo-Silikat-Glaskeramiken (LAS-Glaskeramik) dar. Bei hierauf basierenden Glaskeramiken kann mittels eines Temperprozesses beliebig zwischen einer klar transparenten Hochquarz-Mischkristallphase und einer opaken Keatit-Phase des Materials gewählt werden, so dass diese besonders geeignet erscheinen. In anderen Worten, es wird eine Glaskeramik, vorzugsweise eine Lithium-Alumo-Silikat-Glaskeramik, erzeugt, bei welcher durch eine gezielte Temperatur-/Zeitbeaufschlagung über die Kristallbildung und das Kristallwachstum die Kristallitgröße und deren Verteilung im Volumen gezielt eingestellt wird, und wobei die Kristallite als Streuzentren in der Glaskeramik wirken.

Derartige glaskeramische Materialien sind beispielsweise unter der Bezeichnung CERAN CLEARTRANS® für Kochfelder oder unter der Bezeichnung ROBAX® für Kaminsichtscheiben von der Firma Schott AG, Mainz, erhältlich. Derartige Glaskeramiken und Herstellverfahren sind auch beispielsweise in der Schrift EP 1 266 543 A1 der Anmelderin genannt, dessen Inhalt hiermit ebenfalls vollumfänglich zum Gegenstand der vorliegenden Erfindung gemacht wird.

Grundsätzlich kann auch ein Röntgen-opakes Glas oder eine entsprechende transparente Glaskeramik für den Diffusor-Grundkörper beziehungsweise für die eingelagerten Streuelemente und/oder die Hülle eingesetzt werden.

Im Hinblick auf Wellenlängenbereich von 0,4 $\mu$m bis etwa 2,2 $\mu$m, zum Beispiel bei einer Verwendung des Beleuchtungssystems für die endovenöse Lasertherapie (EVLT), können auch spezielle IR-transparente Gläser, wie sie beispielsweise unten den Bezeichnungen N-PK52a, ein Phosphat-Kron-Glas, oder IRG7, ein Blei-Silikat-Glas, alle erhältlich bei der Firma Schott AG, Mainz, zum Einsatz kommen.

Eine nachträgliche Beeinflussung der Streuzentren im Diffusor-Grundkörper hinsichtlich ihrer Streuwirkung kann auch erreicht werden, wenn der Diffusor-Grundkörper nach einer nachträglichen Temperung, insbesondere einer Gradiententemperung, unterzogen wird. So lässt sich beispielsweise ein Entmischungssprozess bei den beispielsweise als Streuzentren eingesetzten Weißglasstäbchen mit einer solchen Gradiententemperung variieren. Bei Glaskeramik-basierten Streuzentren kann die Kristallbildung und das Kristallwachstum respektive die Kristallitgröße und deren Verteilung im Volumen des Diffusor-Grundkörpers beeinflusst werden.

Ein auf diese Weise erhaltener Diffusor-Grundkörper kann dann mittels Spleißen oder Verkleben mit einem brechwertangepassten Kleber mit dem Lichtleiter verbunden werden.

Um unerwünschte Streuung, Streulichteffekte und/oder Lichtreflexe insbesondere am Übergangsbereich von Lichtleiter und Diffusor-Grundkörper zu reduzieren, kann in einer weiteren bevorzugten Ausführungsvariante vorgesehen sein, dass die Streuzentren in unmittelbarer Nähe zu der Einkoppelfläche des Diffusor-Grundkörpers eine gegenüber der Streuwirkung im übrigen Bereich des Diffusor-Grundkörpers reduzierte Streuwirkung aufweisen.

Dies kann beispielsweise durch eine zusätzliche Temperaturbeaufschlagung, zum Beispiel während des Spleißvorganges von Diffusor-Grundkörper und Lichtleiter, erzielt werden. Dadurch kann beispielsweise lokal eine bei Streuelementen aus Weißglas vorhandene Entmischung (beispielsweise Phasenseparation, Entglasung) zumindest teilweise verändert, bspw. auch reduziert oder wieder rückgängig gemacht werden. Durch Letzteres nimmt die Streuwirkung in diesem Bereich ab.

Bei all den zuvor aufgeführten Materialien und Verfahren zum Herstellen eines Diffusor-Grundkörpers kann mittels bestimmter Temperatur-/Zeit-Führung eine wellenlängenabhängige Streuwirkung erzielt werden, was im Wesentlichen dadurch geschieht, dass die Größe der Streuzentren damit beeinflusst werden kann. Damit kann, je nach Anwendungswellenlänge bzw. Anwendungswellenlängenbereich eine optimale Streuwirkung erzielt werden, so dass Anforderungen beispielsweise zur Homogenität der Abstrahlung erfüllt werden können. Die Abstrahlcharakteristik ist damit gezielt einstellbar.

Darüber hinaus sind weitere nachgeschaltete Prozesse denkbar, bei denen der Intensitätsverlauf der seitlichen Abstrahlung des Diffusor-Grundkörper korrigiert beziehungsweise angepasst werden kann. Diese umfassen insbesondere Verfahren, die einerseits im Volumen und/oder den Oberflächen eines Materials dessen Eigenschaften beispielsweise dessen Brechwert, und/oder Zusammensetzung, zum Beispiel als kolloidale Ausscheidungen und/oder Keimbildung und/oder Kristallisation, zumindest lokal modifizieren können und/oder andererseits materialab- oder -auftragende Modifikationen in nahezu beliebiger geometrischer Form und Anordnung ermöglichen.

Hierzu gehören beispielsweise Verfahren der Laserbearbeitung, die zum Beispiel mittels Kurzpuls- oder $CO_2$-Lasern das Einbringen von Brechwertveränderungen oder Erzeugen von Strukturen im Volumen, zum Beispiel Hohlräume, und/oder den Oberflächen einbringen können.

Des Weiteren sind Druckprozesse zum Aufbringen beziehungsweise Herstellen von Strukturen, beispielsweise einer Rasterverlaufsstruktur auf der Oberfläche des Diffusor-Elementes und/oder des Diffusor-Grundkörpers und/oder der Hülle, zum Beispiel mittels druckbarer organischer oder keramischer Farben mit entsprechenden Pigmenten oder mittels einer Glasfluß-basierten Farbe, fallweise mit entsprechender thermischer Nachbehandlung, anwendbar. Ebenfalls durchführbar sind photolithografische Verfahren und Prozessschritte, wie sie beispielsweise im Speziellen für eine Volumen- oder Oberflächenstrukturierung zum Beispiel photosensitiver oder photostrukturierbarer Gläser und Glaskeramiken eingesetzt werden.

Ebenso möglich sind gegebenenfalls selektives naß- oder trockenchemisches Ätzen des Diffusor-Grundkörpers und/ oder des Diffusor-Elementes an deren Oberflächen, wobei auch hier photolithografische Prozeßschritte zur Anwendung kommen können. Auch mechanisch und/oder abrasiv wirkende Verfahren können zur Strukturierung, insbesondere Aufrauhung einer Oberfläche des Diffusor-Elementes und/oder des Diffusor-Grundkörpers und/oder der Hülle, beispielsweise Schleifen, Läppen oder Sandstrahlen, eingesetzt werden.

Die vorgeschlagenen beispielhaften Methoden oder Verfahren können auch kombiniert angewandt werden. Die so erzeugbaren Diffusor-Elemente und/oder der Diffusor-Grundkörper und/oder Hüllen weisen somit zumindest teilweise oder abschnittsweise in deren Volumen und/oder auf deren Oberflächen Strukturen auf.

In einer weiteren vorteilhaften Ausführungsvariante kann vorgesehen sein, dass der Diffusor-Grundkörper zumindest teil- und/oder abschnittsweise eine Beschichtung mit streuenden Partikeln und/oder der Diffusor-Grundkörper zumindest teil- und/oder abschnittsweise eine zusätzliche, weitere Ummantelung aus einem eingefärbten Glas oder einem eingefärbten Kunststoff aufweist. Beispiele für eine derartige Beschichtung, welche zusätzliche eine Lambert'sche-Abstrahlcharakteristik unterstützt und hierbei insbesondere eine in Lichteinkopplungsrichtung vorwärts gerichtete Abstrahlung reduziert, ist eine Beschichtung mit Bornitirid (BN). Weitere Beschichtungen dieser Art können zum Beispiel aus Titanoxid, Kalziumkarbonat oder Zirkonoxid bestehen.

Die zusätzliche Ummantelung kann beispielsweise aus Weißglas ausgeführt sein, welches in dessen Glasmatrix streuende Elemente enthält. Im Übergangsbereich, im Bereich der Verbindungsstelle beziehungsweise im Bereich des Zwischenmediums zwischen Diffusor-Grundkörper und Lichtleiter kann als zusätzlich Ummantelung zum Beispiel ein Farbglasrohr vorgesehen sein, wobei die Einfärbung und deren Intensität so gewählt sein kann, dass insbesondere die verwendete Wellenlänge des Lichtes unterdrückt oder gar blockiert wird.

Damit können ungewollte Reflexe und damit ungewollte Abstrahlungen unterdrückt werden. Entsprechende Ummantelungen aus Kunststoff sind beispielsweise eingefärbte Silikon- oder PTFE bzw. FEP-Schläuche. Vorteilhaft sind auch entsprechende Tauchbeschichtungen aus Silikon oder entsprechenden Kunststoffen oder Lacken einsetzbar.

Bei all den zuvor ausgeführten Ausführungsbeispielen ist im Hinblick auf eine möglichst geringe Erwärmung des in den Körper applizierten Bauteils, insbesondere der nachfolgend genannten Komponenten des Bauteils darauf zu achten, dass das Diffusor-Element und/oder der Diffusor-Grundkörper und/oder die Hülle eine niedrige Absorption bei der jeweiligen Anwendungswellenlänge aufweist. Hier sind insbesondere Temperaturerhöhungen über 42 °C aus medizintechnischer Sicht zu vermeiden. Dies ist umso wichtiger, wenn beispielsweise für bestimmte Therapiearten der Diffusor mit höheren Laser-Leistungen betrieben wird. Es sei denn, es handelt sich um Therapien, die insbesondere auf einer Erwärmung des Gewebes abzielen (z.B. LITT).

Die Herstellung eines erfindungsgemäßen Diffusor-Grundkörpers mit einem dem Anwendungszweck angepassten Beleuchtungsprofil, insbesondere der Homogenität der Intensität der im Betriebszustand gewünschten radialen, sphärischen Abstrahlcharakteristik, stellt hohe Anforderungen an verschiedene Prozessschritte. Daher ist das Herstellungsverfahren eines erfindungsgemäßen Diffusor-Grundkörpers ebenfalls ein wichtiger weiterer Aspekt der vorliegenden Erfindung.

Vor oder nach dem Verbinden des Diffusor-Grundkörpers mit dem Lichtleiter kann ein Tauchprozess vorsehen sein, um eine Hülle zu erzeugen, welche vorzugsweise den Diffusor-Grundkörper komplett umschließt. Da es vorteilhaft für die Haltbarkeit der Verbindungsstelle ist, wenn diese auch durch eine Hülle geschützt wird, erfolgt das Tauchen vorteilhaft erst nachdem der Diffusor-Grundkörpers mit dem Lichtleiter verbunden wurde. Auf diese Weise ist es möglich, dass auch die Verbindungszone zwischen Diffusor-Grundkörper und Lichtleiter, z.B. der Spleißbereich umschlossen wird.

Die Hülle kann dabei, wie oben erläutert, eine für seitlich aus dem Diffusor-Element emittiertes Licht transparente oder transluzente Schicht, bestehend aus Flüssig-Silikon, thermoplastischem Polymer, Schmelzkleber, 2-komponentigen Kleber oder Sol-Gel-Glas, einen Schrumpfschlauch oder auch zusätzlich aufgesetzte transparente oder transluzente Aufsatzelementen umfassen, die den Diffusor-Grundkörper und/oder die Übergangsstelle zwischen Diffusor-Grundkörper und Lichtleiter zumindest teil- oder abschnittsweise oder auch vollständig umschließt.

Bei einer LAS-Glaskeramik kann beispielsweise durch die Temperaturführung während der Keramisierung aus einer ursprünglich klar transparenten Hochquarz-Mischkristallphase eine Kristallumwandlung in eine opake Keatit-Phase herbeigeführt werden, welche die Streuzentren darstellen können.

Eine bevorzugte Verwendung des Beleuchtungssystems, wie es zuvor in seinen verschiedenen Ausführungsvarianten beschrieben wurde, sieht den Einsatz für eine photodynamische Therapie (PDT) beispielsweise zur Tumortherapie, z.B. im Gehirn, in der Lunge, in den Atemwegen, in der Blase oder in der Gebärmutter, vor. Therapeutische Verwendung des Systems ist kein Teil der Erfindung.

Ebenso denkbar ist der Einsatz für eine endovenöse Lasertherapie (EVLT), beispielsweise zur Behandlung von Krampfadern, für eine Laser-induzierte interstitielle Thermotherapie (LITT) oder für Anwendungen im Bereich der Dentalmedizin, für eine Blasen- oder Prostata-Behandlung, für eine lichtinduzierte Behandlung von Entzündungen im Hals-/Rachenraum (Mukositis), Augenheilkunde sowie Dermatologie, wie diese eingangs beschrieben wurden. Im Bereich Dentalmedizin sind hier insbesondere Applikationen zur Wund- oder Parodontose-Behandlung zu nennen, wobei mittels derartiger Diffusoren Zahntaschen entsprechend effizient ausgeleuchtet werden können. In der Augenheilkunde können damit Sonden bereitgestellt werden, mit denen bei Eingriffen im Bereich der Netzhaut diese in den Augapfel eingestochen werden können, so dass große Bereiche der Netzhaut gleichmäßig ausgeleuchtet werden können. Darüber hinaus gibt es Anwendungen in der Hirnforschung, bei denen mittels Licht einzelnen Gehirnbereich stimuliert und damit Krankheitssymptome behandelt werden können.

Eine weitere Verwendung des Beleuchtungssystems, wie es zuvor in seinen verschiedenen Ausführungsvarianten beschrieben wurde, sieht den Einsatz für eine photodynamische Therapie (PDT) bzw. Photo-Immuno-Therapie (PIT) zur Tumortherapie vor, wobei zumindest ein Lichtleiter mit dem Diffusor-Element aus anderen Diffusor-Elementen abgestrahltes Licht aufnimmt und über den Lichtleiter einem Detektor zur spektroskopischen Analyse weiterleitet. Dabei werden dem Patienten neben den verschiedenen Lichtaussendenden Diffusor-Lichtleitern auch lichtempfangende Diffusor-Lichtleiter appliziert, wobei anhand der spektralen Unterschiede zwischen eingekoppeltem und empfangenen Licht auf ein Ansprechen der PDT-Behandlung geschlossen werden kann (siehe dazu Finlay et al., Proc. SPIE Int. Soc. Opt. Eng. 2014, June 14; 5315: Page 132-142). PIT-Behandlungen sind u.a. in "Study of RM-1929 and Photoimmunotherapy in Patients With Recurrent Head and Neck Cancer", US National Library Medicine, beschrieben.

Im Hinblick auf Dosimetrie-Messungen bei der PDT- bzw. PIT-Behandlung können auch die zuvor beschriebenen Diffusor-Elemente zum Detektieren des vom Gewebe zurück gestreuten Lichts verwendet werden. Damit können zum einen spektroskopische Untersuchungen durchgeführt werden, wenn insbesondere das zurück gestreute Licht bei zum eingestrahlten Licht unterschiedlichen Wellenlängen analysiert wird. Damit kann beispielsweise detektiert werden, inwieweit der Photosensitizer vollständig reagiert hat. Andererseits können Intensitätsmessungen auch Aufschluss geben, inwieweit die Bestrahlung des tumorbefallenen Gewebes ausreichend ist bzw. nicht überstrahlt wird.

Darüber hinaus sind auch Anwendungen im industriellen Bereich vorteilhaft, etwa zur Inspektion von schwer zugänglichen Stellen beispielsweise an oder in einer Maschine, bei denen es insbesondere auf eine homogene Ausleuchtung ankommt, oder auch spektroskopische Anwendungen oder in der Biochemie, bei der durch Licht biochemische In-Vitro-Reaktionen stimuliert werden.

Die Erfindung wird im Folgenden anhand eines in den Figuren dargestellten Ausführungsbeispiels näher erläutert. Es zeigen:

Fig. 1            schematisch ein Beleuchtungssystem mit einem Lichtleiter und einem Diffusor-Element in einer PDT-Anwendung,
Fig. 2a bis 2c    eine schematische Darstellung verschiedener Geometrie-Ausprägungen des Diffusor-Elements,
Fig. 3            eine detaillierte schematische Darstellung eines Querschnitts durch ein Diffusor-Element mit Lichtleiter,
Fig. 4            ein Verlaufsdiagramm winkelabhängiger Intensitätsverläufe,
Fig. 5            ein weiteres Verlaufsdiagramm eines Intensitätsverlaufs und eine jeweilige spezifische Streulänge für zwei verschieden streuende Diffusor-Grundkörper,
Fig. 6            schematisch ein Diffusor-Element, welches als Ballon-Katheter ausgebildet ist,
Fig. 7a bis 7c    Diffusor-Elemente mit unterschiedlichen Längen und
Fig. 8            ein weiteres Verlaufsdiagramm winkelabhängiger Intensitätsverläufe zu den in den Figuren 7a bis 7c gezeigten Diffusor-Elementen inkl. einer schematischen Darstellung des Messprinzips,
Fig. 9            ein weiteres Verlaufsdiagramm eines sphärischen Diffusors, welcher als Kugel ausgebildet ist und bei dem das Diffusor-Material unterschiedlich thermisch vorbehandelt wurde,
Fig. 10           einen im Wesentlichen als Kugel ausgebildeten Diffusor,
Fig. 11           eine SEM-Aufnahme vom Querschnitt des als Kugel ausgebildeten Diffusors im Zentrum der Kugel und
Fig. 12           eine weitere SEM-Aufnahme vom Querschnitt des Diffusors in einer oberflächennahen Zone.

Detaillierte Beschreibung bevorzugter Ausführungsformen

Bei der nachfolgenden Beschreibung der detaillierten Ausführungsformen bezeichnen gleiche Bezugszeichen in den beiliegenden Figuren jeweils gleiche oder gleich wirkende Bestandteile.

Zum besseren Verständnis werden die nachfolgenden Definitionen vorgenommen.

Im Sinne der vorliegenden Offenbarung umfasst der Begriff des Beleuchtungssystems Beleuchtungsvorrichtungen und insbesondere Beleuchtungsvorrichtungen, welche zur Anwendung für medizintechnische Zwecke geeignet und insbesondere, soweit diese mit lebendem Gewebe in Kontakt treten sollen, zumindest abschnittsweise desinfizierbar und/oder sterilisierbar sind.

Die Aussage "für ein medizintechnisches Therapie- und/oder Diagnosesystem" umfasst auch die Verwendung oder Anwendung des hier offenbarten Beleuchtungssystems selbst als medizinisches Therapie- und/oder Diagnosesystem.

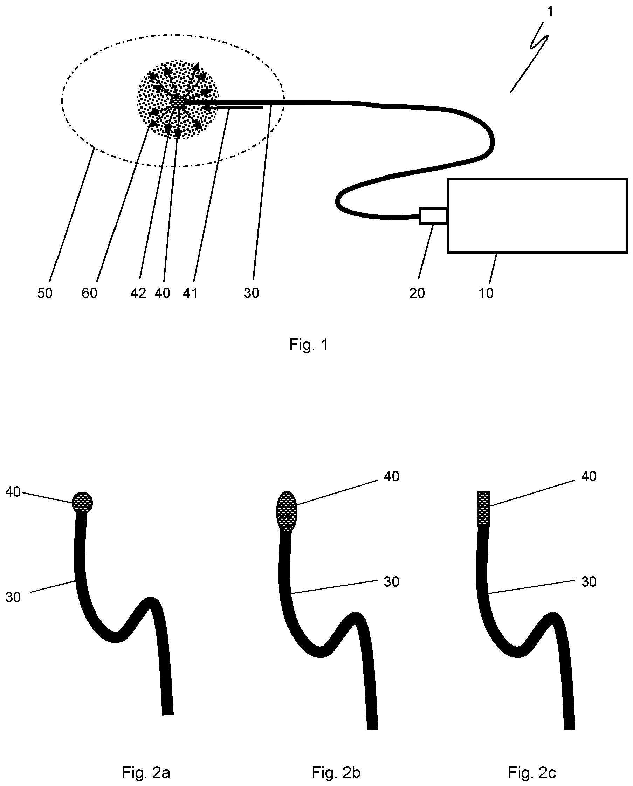

Fig. 1 zeigt schematisch den Aufbau eines Beleuchtungssystems 1 gemäß der einer bevorzugten Ausführungsform der Erfindung. Beispielhaft ist hier eine medizintechnische PDT-Anwendung dargestellt.

Im gezeigten Beispiel umfasst das Beleuchtungssystem 1 eine Laser-Lichtquelle 10, welche im Betriebszustand Licht in einem bestimmten Spektralbereich aussendet. Für PDT-Anwendungen, wie sie eingangs beschrieben sind, werden Laser verwendet, die auf den zuvor verabreichten biochemisch modifizierten Farbstoff (Photosensitizer) abgestimmte Wellenlänge üblicherweise im sichtbaren Bereich, beispielsweise im grünen Spektralbereich bei 532 nm oder im roten Spektralbereich bei zum Beispiel 690 nm, aussenden.

Ein Lichtleiter 30 ist mit einem Stecker 20 an seinem proximalen Ende an die Laser-Lichtquelle 10 angeschlossen. Als proximales Ende wird hierbei das Ende des Lichtleiters 30 bezeichnet, in welches Licht eingekoppelt wird. Am distalen Ende weist der Lichtleiter 30 ein Diffusor-Element 40 auf, welches direkt oder ggf. über hier nicht dargestellte Kanülen in ein Tumor-Gewebe 60 eingebracht wird, welches sich innerhalb eines gesunden Gewebes 50 gebildet hat. Als distales Ende wird hierbei das andere Ende des Lichtleiters 30 bezeichnet, welches in der Regel zu dem proximalen Ende des Lichtleiters 30 entfernt angeordnet ist und aus welchem insbesondere Licht austritt.

Die Laserstrahlung gelangt dabei über eine Lichteinkopplung 41 am Diffusor-Element 40 in das Diffusor-Element 40 und wird in diesem mehrfach gestreut und über die Oberfläche des Diffusor-Elements 40 im Wesentlichen radial sphärisch abgestrahlt. Im dargestellten Beispiel ist die Lichtauskopplung anhand rein beispielhafter Strahlen 42 gezeigt. Dabei kommt es auf eine möglichst homogene Abstrahlung in eine das Diffusor-Element 40 umgebende Kugelsphäre an. Insbesondere sind Intensitätsspitzen zu vermeiden. Durch eine photoinduzierte biochemische Reaktion, wie sie eingangs beschrieben ist, kommt es nach der Behandlung idealerweise zu einem Absterben des Tumor-Gewebes 60.

In der Regel werden als Lichtleiter 30 Quarz-Fasern verwendet, wobei die Stecker 20 in der Regel als koaxialer Steckverbinder, sogenannte SMA-Stecker, ausgebildet sind, bei denen die Fasern in den Stecker 20 geklebt sind. Vorteilhaft hinsichtlich der thermischen Belastbarkeit können auch Stecker 20 mit Neusilber-Hülsen sein, bei denen der Lichtleiter 30 in die Neusilber-Hülse formschlüssig durch plastische Verformung eingebracht, gecrimpt, ist. Darüber hinaus können bei größeren Laser-Leistungen auch Stecker 20 zum Einsatz kommen, bei denen das Faserende des Lichtleiters 30 durch ein Kegelprisma geschützt ist, was vorteilhaft bei Fehljustagen sein kann.

Die Figuren 2a bis 2c zeigen beispielhaft in schematischer Darstellung verschiedene bevorzugte Geometrie-Ausprägungen für ein derart im Wesentlichen sphärisch abstrahlendes Diffusor-Elements 40. Fig. 2a zeigt ein Diffusor-Element 40, welches eine im Wesentlichen kugelförmige Geometrie aufweist. Fig. 2b zeigt ein Beispiel für ein Diffusor-Element 40 welches als Ellipsoid ausgebildet ist. Fig. 2c zeigt ein Beispiel, bei dem das Diffusor-Element 40 als kurzer Zylinderabschnitt ausgebildet ist. Hierbei sei angemerkt, dass auch andere, z.B. tropfenförmige, ovale Geometrien, oder Geometrien, welche sich als Überlagerung oder Kombination von o.g. geometrischen Grundformen darstellen lassen, hierbei eingeschlossen sind. Denkbar sind auch kuppenförmige Geometrien. Kennzeichnend ist hier die eher geringe Gesamtlänge des Diffusor-Elements 40 in Bezug auf den äußeren Durchmesser des Lichtleiters 30. Üblicherweise beträgt dieser für die zum Einsatz kommenden Quarzfasern zwischen 300 $\mu$m und 800 $\mu$m, so dass typische Längen des Diffusor-Elementes 40 gemäß der Erfindung demnach zwischen 200 $\mu$m und max. 10 mm betragen.

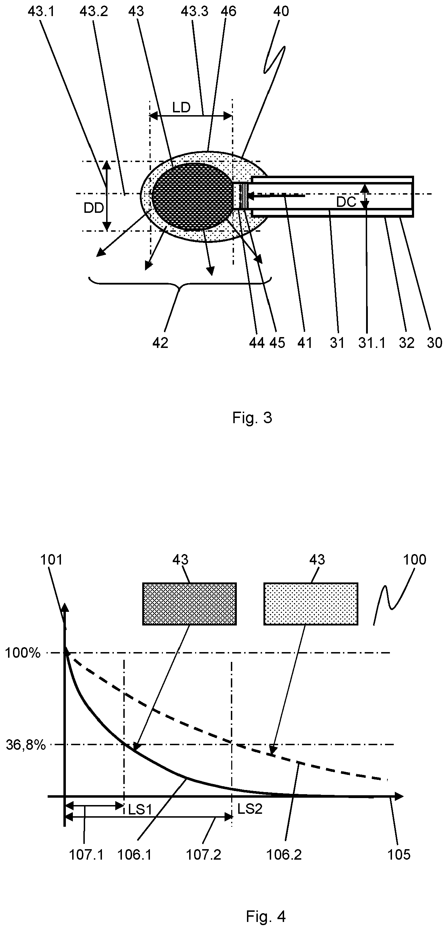

Figur 3 zeigt in einer detaillierten schematischen Darstellung einen Querschnitt durch ein Diffusor-Element 40 mit Lichtleiter 30.

Die Befestigung von Diffusor-Grundkörper 43 und Lichtleiter 30 erfolgt innerhalb der Verbindungszone 44 durch beispielsweise einen Spleiß- oder Verklebe-Prozess mit einem brechwertangepassten hochtransparenten Kleber. Beim Spleißen wird mittels einer Corona-Entladung und/oder mittels eines Lasers, üblicherweise mit einem $CO_2$-Laser, der Lichtleiter 30 und der Diffusor-Grundkörper 43 anbeziehungsweise aufgeschmolzen und zusammengeführt. Je nach Material, welches für den Diffusor-Grundkörper 43 und dem Lichtleiter 30 verwendet wird, kann es erforderlich sein, dass zur Anpassung derer Wärmeausdehnungskoeffizienten ein Zwischenmedium 45 verwendet wird. Dieses kann beispielsweise bei einer Glas-/Quarz-Verschmelzung ein Lot- oder Übergangsglas oder ein optischer Kleber oder Kitt sein.

Der Lichtleiter 30 besteht bei den zuvor beschriebenen Anwendungen meist aus Quarzglas mit einem Kern 31 mit einem Brechungsindex $n_1$ und einem Kerndurchmesser DC 31.1 von üblicherweise zwischen 200 und 800 $\mu$m sowie einem Mantel 32 mit einem Brechungsindex $n_2$, wobei gilt $n_1 > n_2$.

Üblicherweise hat eine derartige Faser noch eine äußere als Buffer bezeichnete Polymerschicht, z.B. bestehend aus Polyamid oder Polyimid. Die damit üblicherweise erzielbare numerische Apertur NA beträgt etwa 0,22. Die Lichteinkopplung 41 erfolgt über eine Einkoppelfläche, welche durch eine Verbindungszone 44 des Diffusor-Grundkörpers 43 ausgebildet ist.

Der Diffusor-Grundkörper 43 kann, wie exemplarisch dargestellt, einen ellipsenförmigen Querschnitt aufweisen. Wie zuvor dargestellt (vergl. Fig. 2a bis 2c), können unterschiedliche Formen auch für den Diffusor-Grundkörper 43 zutreffen. Geometrisch kennzeichnend sind der Durchmesser DD 43.1 des Diffusor-Grundkörpers 43, wobei hierunter eine Ausdehnung in einer Richtung senkrecht zur Richtung der Lichteinkopplung gemeint ist, also senkrecht zur Längsrichtung des Lichtleiters im Bereich der Einkoppelung, sowie die Diffusorlänge LD 43.3 entlang seiner Längsachse 43.2, sofern dieser z.B. eher oval oder elliptisch ausgeprägt ist, also parallel zur Richtung der Lichteinkopplung bzw. in einer Richtung parallel zur Längsrichtung des Lichtleiters im Bereich der Einkoppelung.

Der Diffusor-Grundkörper 43 besteht gemäß der Erfindung, wie zuvor beschrieben aus einem anorganischen Material, insbesondere aus einem Glas, einer Glaskeramik oder einem glasähnlichen Werkstoff, in welchem aufgrund seiner Zusammensetzung fein verteilte Streuzentren mit einer bestimmten Größenverteilung gezielt ausbildbar sind. Hierfür eignen sich insbesondere die zuvor beschriebenen Materialansätze.

Zum Schutz ist der Diffusor-Grundkörper 43 mit einer Hülle 46 versehen, die den Diffusor-Grundkörper 43 komplett umschließt, wobei es vorteilhaft sein kann, wenn zusätzlich auch die Verbindungszone 44 zwischen Diffusor-Grundkörper 43 und Lichtleiter 30 umschlossen ist. Üblicherweise weist der Lichtleiter 30 in diesem Bereich keine Buffer-Schicht auf (in Fig. 3 dargestellt ohne Mantel 32) auf, so dass diese Zone mit einer derartigen Hülle 46 geschützt werden kann, was sonst mit einem sogenannten Recoating-Prozess zum mechanischen Schutz erfolgen müsste.

Die Hülle 46 kann dabei als eine für seitlich aus dem Diffusor-Element emittiertes Licht transparente oder transluzente Schicht, bestehend aus Flüssig-Silikon, thermoplastischem Polymer, Schmelzkleber, 2-komponentigen Kleber oder Sol-Gel-Glas, aus einer Lackschicht, aus einem Schrumpfschlauch oder aus zusätzlich aufgesetzten transparenten oder transluzenten Aufsatzelementen bestehen, die den Diffusor-Grundkörper 43 und die Übergangsstelle zwischen Diffusor-Grundkörper 43 und Lichtleiter 30 umschließen.

Denkbar ist auch ein Einschluss mit einem vergleichsweise niedrig schmelzenden Glas, wobei hier aber darauf zu achten ist, dass bei dem Aufbringen des niedrig schmelzenden Glases bei erhöhter Temperatur nicht in die Abstrahlcharakteristik des Diffusor-Elements ungünstig eingegriffen wird.

In vorteilhafter Ausgestaltung ist der Durchmesser DD 43.1 des Diffusor-Grundkörper 43 grösser als der Kerndurchmesser 31.1 oder Faserbündeldurchmesser 31.1 des Lichtleiters 30 ausgelegt, so dass das Licht optimal in den Diffusor-Grundkörper 43 eingekoppelt wird. Andererseits können damit Montage und die Justage von Lichtleiter 30 und Diffusor-Grundkörper 43 erleichtert und/oder Montagetoleranzen ausgeglichen werden. Zudem kann erreicht werden, dass noch eine gewisse Lichtausbreitung rückwärts gerichtet, also in eine Richtung der Lichteinleitung durch den Lichtleiter, erzielt werden kann.

Fig. 4 beschreibt in einem Verlaufsdiagramm 100 einen Intensitätsverlauf für zwei Diffusor-Grundkörper 43, welche zur empirischen Bestimmung einer spezifischen Streulänge 107.1, 107.2 als stabförmiges Material ausgebildet wurden. Dargestellt sind in einem ersten Intensitätsverlauf 106.1 und in einem zweiten Intensitätsverlauf 106.2 die senkrecht zur Stabachse gemessene Intensitäten 101 in Abhängigkeit zum Abstand zur Verbindungszone $I_{(x)}$ 44 bzw. Licht-Einkoppelstelle, welche sich im Verlaufsdiagramm 100 auf der linken Seite befindet, wobei direkt an der Licht-Einkoppelstelle die Intensitätsverläufe 106.1, 106.2 auf 100% normiert sind. In beiden Fällen nimmt die Intensität 101 annähernd gemäß einer Expontialfunktion gemäß folgender Beziehung ab, sofern die Streuwirkung über die Länge der Stäbe konstant ist:

$$I_{(X)} = I_0 * \exp (- X/LS) \tag{1}$$

wobei $I_0$ die Anfangsintensität an der Licht-Einkoppelstelle darstellt und $I_{(x)}$ die gemessene Intensitäten 101 in Abhängigkeit zum Abstand zur Verbindungszone $I_{(x)}$ 44 ist.

Die spezifische Streulänge LS1, LS2 107.1, 107.2 ist dabei definiert als die Entfernung von der Licht-Einkoppelstelle, bei der die Lichtintensität auf den Wert 1/e bzw. auf 36,8% abgefallen ist. Exemplarisch dargestellt ist ein Diffusor-Grundkörper 43 mit einer vergleichsweise intensiven Lichtstreuung (linker Diffusor-Grundkörper 43), was sich in einem steilen Intensitätsverlauf 106.1 und einer dazu korrespondierenden vergleichsweise kleinen Streulänge LS1 107.1 äußert. Der rechts dargestellte Diffusor-Grundkörper 43 zeigt eine weniger intensive Streuwirkung, was sich in einem eher flacheren Intensitätsverlauf 106.2 und einer dazu korrespondierenden vergleichsweise größeren Streulänge LS2 107.2 äußert. Durch gezielte Temperatur/Zeit-Prozesse kann erfindungsgemäß das Material der Diffusor-Grundkörper 43 hinsichtlich seiner Streueigenschaften eingestellt werden, wie dies bereits beschrieben wurde, und mit einer derartigen Messung quantitativ charakterisiert werden.

Um eine ideale radial gleichmäßige Intensitätsverteilung zu erzielen, ergeben sich Anforderungen an die Geometrie, insbesondere an den Durchmesser des Diffusors DD 43.1 sowie an die Diffusorlänge DL 43.3 in Abhängigkeit von den StreuEigenschaften des Diffusor-Grundkörpers 43, welche sich zumindest in erster Näherung empirisch durch die spezifische Streulänge LS1, LS2 107.1, 107.2 beschreiben lassen. Hierbei spielt auch der Kerndurchmesser DC 31.1 des Lichtleiters 30 bzw. ein Faserbündeldurchmesser eine gewisse Rolle. Dabei haben sich folgende Geometrieverhältnisse als zielführend erwiesen:

Die radiale, sphärische Abstrahlcharakteristik wird weiterhin begünstigt durch eine Ausdehnung des Diffusor-Grundkörpers, wobei die größte Ausdehnung LD des Diffusor-Grundkörpers in einer ersten Richtung nicht größer ist als das 10-fache, bevorzugt das 5-fache und besonders bevorzugt das 2,5-fache der Ausdehnung des Diffusor-Grundkörpers in einer zweiten Richtung DD senkrecht zu dieser ersten Richtung, bevorzugt nicht größer als das 2-fache und besonders bevorzugt nicht größer als das 1,5-fache.

In einer besonders bevorzugten Ausführungsform ist die Ausdehnung des Diffusor-Grundkörpers in einer ersten Richtung gleich der Ausdehnung des Diffusor-Grundkörpers in einer zweiten Richtung senkrecht zu dieser ersten Richtung, wodurch eine kugelförmige Ausbildung gegeben ist.

Der äußeren Durchmesser des Lichtleiters liegt üblicherweise für die zum Einsatz kommenden Quarzfasern zwischen 200 μm und 800 μm, so dass typische Ausdehnungen des Diffusor-Elementes in einer bevorzugten Ausführungsform der Erfindung zwischen 300 μm und 3 mm betragen können. Die maximale Ausdehnung des Diffusor-Grundkörpers LD in einer Richtung liegt in einem Bereich zwischen 200 μm und 10 mm, bevorzugt zwischen 250 μm und 4 mm, und besonders bevorzugt zwischen 300 μm und 3 mm.

Bevorzugt gilt weiterhin für den Kerndurchmesser DC:

$$DD \geq DC \qquad\qquad\qquad (2)$$

wobei typischerweise DC im Bereich 200 μm bis 500 μm, bevorzugt zwischen 300 μm und 400 μm liegt. Das Verhältnis des Kerndurchmessers DC 31.1 oder Faserbündeldurchmessers des Lichtleiters 30 und des Durchmessers des Diffusor-Grundkörpers OD 43.1 beträgt damit vorteilhaft ≤ 1,0, bevorzugt zwischen 1,0 und 0,8. Je nach gewünschter Abstrahlcharakteristik kann auch ein Verhältnis von ≤ 0,8 vorgesehen sein.

Für den Durchmesser eines als Kugel ausgebildeten Diffusor-Grundkörpers 43 bzw. für die Diffusorlänge LD 43.3 eines eher länglichen Diffusor-Grundkörpers 43 ergeben sich folgende Geometriebedingungen, wobei sich als günstiger Wert für LS herausgestellt hat, wenn LS in etwa der Länge des Diffusor-Grundkörpers LD 43.3 entspricht:

$$LD \leq LS \quad und \qquad\qquad\qquad (3)$$

$$LD \leq 3 * DC, \ bevorzugt \ LD \leq 2 * DC \qquad\qquad (4)$$

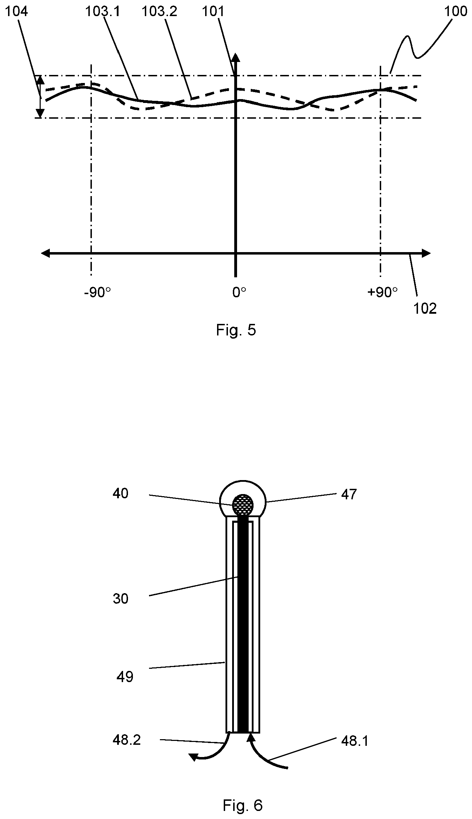

Als Ergebnis einer solchen Geometrieauswahl ergibt das in Fig. 5 gezeigte Verlaufsdiagramm 100, bei dem die Intensität 101 in Abhängigkeit eines Beobachtungswinkels 102 in äquidistantem Abstand um das Diffusor-Element 40 dargestellt ist. Ein erster Intensitätsverlauf 103.1 zeigt eine Intensitätsmessung in einer waagrechten Ebene um das Diffusor-Element 40. Ein zweiter Intensitätsverlauf 103.2 zeigt das Ergebnis einer Intensitätsmessung in einer zu ersten waagrechten Ebenen orthogonal stehenden senkrechten Ebene um das Diffusor-Element 40. Wie zu erkennen ist, liegen beide Intensitätsverläufe 103.1 und 103.2 in einem engen Intensitätstoleranzband 104. Typisch erzielbare Werte dafür liegen unter ± 20 %, bevorzugt unter ± 15 % und besonders bevorzugt unter ± 10 %.

Sind die o.g. Geometrieverhältnisse nicht optimal gewählt, käme es im Bereich um 0°, also in Vorwärtsrichtung der Lichtausbreitung gesehen, zu einer deutlichen Überhöhung des Intensitätsverlaufs 103.1, 103.2, wenn z.B. die spezifische Streulänge LS signifikant größer ist als die Diffusorlänge LD 43.3 bzw. der Durchmesser des Diffusor-Grundkörpers 43 bei einer eher kugelförmigen Geometrie. Dem gegenüber wäre bei etwa 0° eine deutliche Delle im Intensitätsverlauf 103.1, 103.2 zu erkennen, wenn die spezifische Streulänge LS signifikant kleiner ist als die Diffusorlänge LD 43.3 bzw. der Durchmesser des Diffusor-Grundkörpers 43 bei einer eher kugelförmigen Geometrie.

Figur 6 zeigt als weiteres Ausführungsbeispiel einen Diffusor welche innerhalb eines Ballon-Katheters integriert ist. Schematisch dargestellt ist das Diffusor-Element 40, wie zuvor beschrieben, angespleißt an einen als Quarzfaser ausgeführten Lichtleiter 30, welcher sich in einem Ballon 47 befindet, der während der Behandlung aufgeblasen werden kann. Insbesondere beim Einsatz höherer Laser-Leistungen kann weiterhin vorgesehen sein, dass über zusätzliche Zu- und Ablaufkanäle (siehe Flüssigkeitszulauf und-ablauf 48.1, 48.2) um bzw. an dem Lichtleiter 30 eine Kühlflüssigkeit, z.B. eine 0,9 prozentige Kochsalzlösung oder Wasser, zirkulieren kann, was den Wärmeeintrag ins Gewebe deutlich reduzieren hilft.

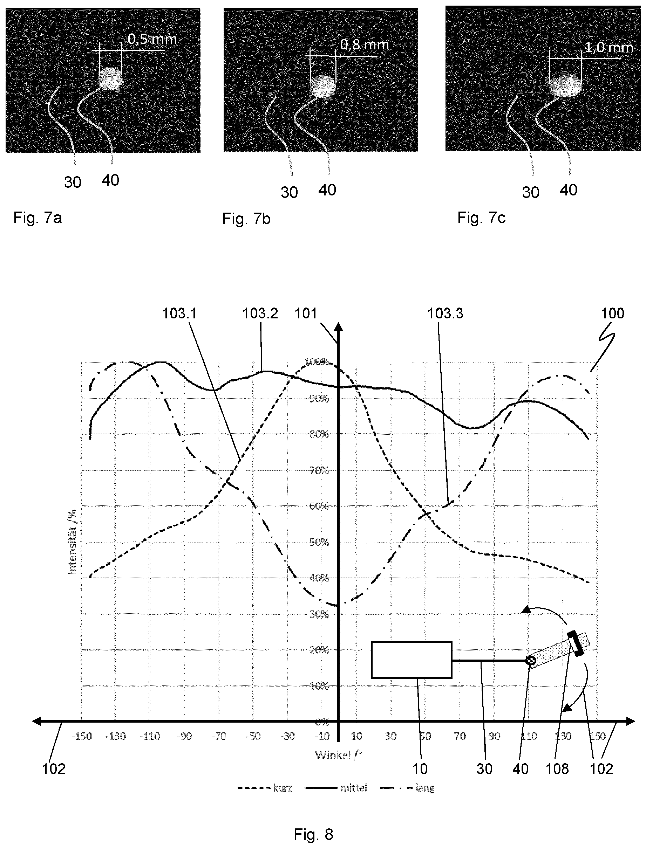

Die Figuren 7a bis 7c zeigen Diffusor-Elemente 40 mit verschiedenen Geometrien. Figur 7a zeigt ein Diffusor-Element 40 dessen Diffusor-Länge 43.3 LD nach einem Verrundungsprozess etwa 0,5 mm beträgt. Figur 7b zeigt ein Diffusor-Element 40 dessen Diffusor-Länge 43.3 LD nach dem Verrundungsprozeß etwa 0,8 mm beträgt und Figur 7c ein Diffusor-Element 40 dessen Diffusor-Länge 43.3 LD nach dem Verrundungsprozess etwa 1,0 mm beträgt.

In einem weiteren Verlaufsdiagramm 100 in Figur 8 sind für die in den Figuren 7a bis 7c gezeigten Diffusor-Elemente 40 entsprechende gemessene Intensitätsverläufe 103.1, 103.2, 103.3 als Azimut-Scan dargestellt, wobei die jeweilige Intensität 101 in Abhängigkeit des Beobachtungswinkels 102 aufgetragen ist. Die schematische Darstellung in der Figur 8 zeigt das Messprinzip der Goniometer-Messung. Das Diffusor-Element 40 wird über eine angespleißte Quarzfaser (Lichtleiter 30) mittels einer Laser-Lichtquelle 10 angestrahlt und gibt das Licht radial/sphärisch ab. Dabei wird ein Detektor 108 in konstantem Abstand, hier ca. 35 mm um den Diffusor-Grundkörper 40 geschwenkt und dabei in Abhängigkeit des Beobachtungswinkels 102 die Intensität 101 gemessen. In Figur 8 sind dabei die Intensitätsverläufe 103.1, 103.2, 103.3 auf die höchste gemessene Intensität 101 normiert (= 100 %). Die Messungen sind mit folgenden Einstellungen bzw. mit folgender Hardware durchgeführt worden:

- Goniometer-Meßbereich: -145° bis +145°
- Abstand Detektor 198 zu Diffusor-Element 40: 35 mm
- GIGAHERTZ OPTIK Optometer P-2000
- Empfindlichkeitsfunktion: radiometrisch 400 nm bis 1.000 nm
- Typ. Empfindlichkeit: 10 nA/(W/m$^2$)

- Öffnungsdurchmesser Detektor (Streuscheibe): 11 mm
- Messöffnung Cosinus-Blickfeld
- Laser-Lichtquelle 10: 4 mW @ 655 nm

Der 1. Intensitätsverlauf 103.1 zeigt die Abstrahlcharakteristik für den in Fig. 7a dargestellten Diffusor-Element 40 mit einer typischen Diffusor-Länge LD 43.3 von 0,5 mm. Hier zeigt sich, dass dieses Diffusor-Element 40 im Vergleich zu dessen charakteristischen Streulänge LS vergleichsweise zu kurz ist. Dies zeigt sich in dem ausgeprägten Intensitätspeak im Bereich um 0°. Der 2. Intensitätsverlauf 103.2 zeigt die Abstrahlcharakteristik für den in Fig. 7b dargestellten Diffusor-Element 40 mit einer typischen Diffusor-Länge LD 43.3 von 0,8 mm. Hier zeigt sich, dass dieses Diffusor-Element 40 im Vergleich zu dessen charakteristischen Streulänge LS hinsichtlich der Diffusor-Länge LD 43.3 angepaßt ist und damit auch eine annähernd gleichmäßige Intensität 101 resultiert. Der 3. Intensitätsverlauf 103.3 zeigt die Abstrahlcharakteristik für den in Fig. 7c dargestellten Diffusor-Element 40 mit einer typ. Diffusor-Länge LD 43.3 von 1,0 mm. Hier zeigt sich, dass dieses Diffusor-Element 40 im Vergleich zu dessen charakteristischen Streulänge LS vergleichsweise zu lang ist. Die Folge ist ein starker Intensitätseinbruch im Bereich um 0°.

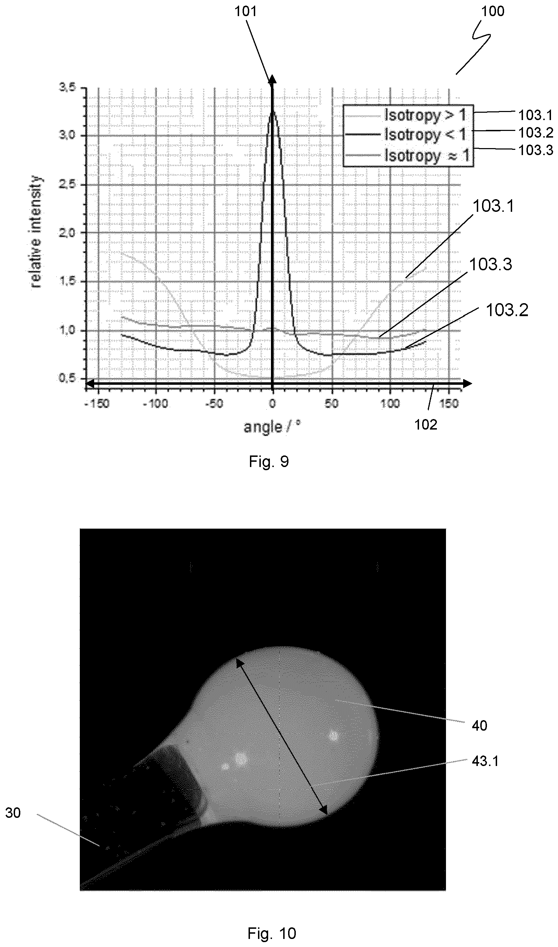

Fig. 9 zeigt ein weiteres Verlaufsdiagramm 100 eines als Kugel ausgebildeten sphärischen Diffusors, bei dem das Diffusor-Material unterschiedlich thermisch vorbehandelt wurde. Aufgetragen sind die Intensitätsverläufe 103.1, 100.2, 100.3 einer relativen Intensität 101 in Abhängigkeit des Beobachtungswinkels 102.

Der 1. Intensitätsverlauf 103.1 zeigt beispielhaft eine mehr seitlich abstrahlende Charakteristik, welche einer Isotropie > 1 entspricht. Hierbei ist das Streuverhalten derart stark eingestellt, dass die in den Diffusor eingekoppelte Strahlung schon recht früh zur Seite gestreut wird und nur ein vergleichsweise geringer Anteil in Vorwärtsrichtung durchgelassen wird.

Der 2. Intensitätsverlauf 103.2 zeigt dagegen einen Verlauf, bei dem nur ein geringer Anteil seitlich gestreut wird und dafür der überwiegende Teil der eingekoppelten Lichtleistung in Vorwärtsrichtung transmittiert wird, was einer Isotropie < 1 entspricht.

Der 3. Intensitätsverlauf 103.3 zeigt ein Beispiel für eine annähernd gleichmäßige Abstrahlung in jede Raumrichtung, was einer Isotropie von ca. 1 entspricht. Die Intensitätsverläufe 103.1 bis 103.3 können durch eine gezielte und überwachte Temperatur-/Zeit-Prozessführung der zuvor beschriebenen Materialien beeinflusst werden.

Im gezeigten Beispiel handelt es sich um das CoralPor™ - Glasmaterial, wie es zuvor beschrieben ist. Die Laser-Wellenlänge beträgt hier 655 nm.

Fig. 10 zeigt den Diffusor in einem Mikroskop-Bild. Das Diffusor-Element 40 ist hier im Wesentlichen als Kugel bzw. kugelförmig ausgebildet und weist einen Diffusor-Durchmesser 43.1 von annähernd 1 mm auf. Der Lichtleiter 30 hat einen Faserkern-Durchmesser von etwa 400 μm, was im gezeigten Beispiel einem Verhältnis Kerndurchmesser des Lichtleiters und Durchmesser des Diffusor-Grundkörpers 43 von etwa 0,4 entspricht.

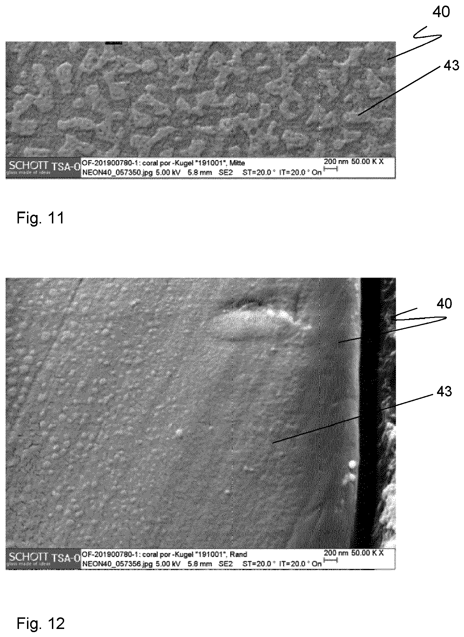

Fig. 11 zeigt in einer SEM-Aufnahme als Schnittbild die Kugelmitte des Diffusor-Grundkörpers 43 des Diffusor-Elementes 40 aus Fig. 10. Dabei wurde der Querschliff vor der SEM-Aufnahmen mit XeF2-Gas angeätzt, um im Querschliff die beiden unterschiedlichen Phasen des Glases zu erkennen. Im Zentrum der Kugel ist ein Durchdringungsgefüge mit Strukturausdehnungen von ca. 200 nm vorhanden, ab mehreren Mikrometern unterhalb der Kugeloberfläche lockert sich diese Struktur zur Oberfläche hin auf, wie dies in Fig. 12 zu erkennen ist, und bildet eine eher amorphe glatte Oberfläche aus.

Zum weiteren mechanischen Schutz und/oder zur Homogenisierung der Abstrahlcharakteristik kann eine weitere Ummantelung 49 aus transparenten und/oder transluzentem, eingefärbten oder farblosen Material (Silikon, Glas oder Quarzglas) vorgesehen sein, die den Diffusor-Grundkörper und/oder die Hülle zumindest teilweise oder abschnittsweise oder vollständig umschließt. Insbesondere kann mit einem transluzenten und/oder Streuzentren enthaltenden Material eine zusätzliche Homogenisierung erzielt werden. Geeignet sind beispielsweise entsprechende Körper oder Schläuche aus Silikon, Teflon oder auch aus einem Polyether-Block-Amid-Block-Copolymer, welches beispielsweise als PEBAX® im Handel bekannt ist. Als Ummantelung 49 haben sich, zumindest abschnittsweise aufgebrachte, dünnwandige Schrumpfschläuchen, z.B. aus PET, erwiesen, welche einlagig oder mehrlagig ausgeführt sein können.

Zwischen Diffusor-Grundkörper 43 und der Ummantelung 49 aus beispielsweise Glas oder Kunststoff kann vorgesehen sein, dass zur Unterdrückung von etwaigen Oberflächenunregelmäßigkeiten, wie beispielsweise Schmutz, Rauigkeit oder ähnlichem auf dem Diffusor-Grundkörper 43, welche das Abstrahlverhalten ungünstig beeinflussen, eine weitere Schicht zwischen Ummantelung 49 und Diffusor-Grundkörper 43 eingebracht ist. Dabei ist auf einen an das Glassystem angepassten Brechungsindex einerseits, auf eine hohe Transparenz sowie auf eine ausreichend hohe Viskosität im Hinblick einer guten Applizierbarkeit zu achten. Als geeignet hat sich bspw. Glyzerin oder Silikone (Öle oder Kleber) als weitere Schicht herausgestellt.

Eine Ausführungsform mit aus Weißglas ausgebildeten Streuelementen 43.6 sieht vor, dass in dieses Weißglas Streuzentren durch Streupartikel gebildet werden, wobei die Konzentrationen der Streupartikel im Streubereich von 10 ppm bis 1.000 ppm und bevorzugt von 20 ppm bis 100 ppm beträgt.

Die Effizienz der Auskopplung aus dem Streubereich, somit dem Volumen des Weißglases der Streustäbe oder des Weißglasrohrs ist neben der streuenden Eigenschaft der Streupartikel als intrinsischem Parameter auch von der Konzentration der Streupartikel im Streubereich selbst abhängig.

Die Konzentrationsangabe in ppm bezieht sich hierbei auf den Anteil der Streupartikel im Verhältnis zu den Masseanteilen der Bestandteile des Weißglases, in welchem die Streupartikel eingelagert sind.

Dienen inhomogene Bereiche des Weißglases als Streuzentren, ergibt sich eine alternative Ausführungsform, in der die inhomogenen Bereiche bevorzugt durch Phasentrennung und/oder Entmischung der Glaskomponenten des Glases gebildet werden, in welches sie eingelagert sind.

Die durch inhomogene Bereiche gebildeten Streuzentren weisen bevorzugt einen Durchmesser eine maximale Abmessung von 10 nm bis 1.000 nm auf, besonders bevorzugt von 100 nm bis 800 nm. Besonders bevorzugt sind diese Streuzentren kugelförmig.

Das Weißglas, in dem die inhomogenen Bereiche als Streuzentren eingelagert sind, kann bevorzugt aus einem As- und Pb-haltigen Silikatglas bestehen. Die Streuzentren weisen in diesem Fall bevorzugt einen gegenüber der umgebenden Glasmatrix erhöhten Gehalt an Pb und/oder As auf.

Alternativ kann das Glas beziehungsweise Weißglas, in welches die inhomogenen Bereiche als Streuzentren eingelagert sind, aus einem Fluor-haltigen Ca-Zn-Silikatglas bestehen. Dann weisen die Streuzentren einen gegenüber der umgebenden Glasmatrix bevorzugt einen erhöhten Gehalt an Fluor auf.

So kann beispielsweise je nach dem eingesetzten Material und den Materialeigenschaften der Streuelemente 43.6 und der dieser umgebenden Matrix 43.4 mittels einer Gradiententemperung erreicht werden, dass durch unterschiedliche Temperaturbeaufschlagung in Richtung der größten Ausdehnung des Diffusors die Streuwirkung beispielsweise stetig variiert werden kann. Damit ist es möglich, dass zum Beispiel für einen direkt an der Einkoppelfläche angeordneten Volumenbereich eine zunächst eher niedrige Streuwirkung und für einen gegenüberliegenden Volumenbereich des Diffusor-Grundkörpers 43 eine eher höhere Streuwirkung einstellbar ist.

Damit können Diffusor-Grundkörper erhalten werden, die Streuelemente mit Streuzentren mit einer Streuzentrendichte pro Volumeneinheit aufweisen, wobei sich die Streuzentrendichte in verschiedenen Volumenbereichen unterscheidet. Ein möglicher Intensitätsabfall in einer Richtung parallel zur Richtung der Einkoppelung des Lichtes somit zumindest teilweise kompensiert werden.

Die vorliegend offenbarte und in den Ansprüchen erwähnte Intensität oder Intensitätsverteilung entspricht in physikalischer Hinsicht auch der Leuchtdichte, welche auch als Luminanz oder Helligkeit bezeichnet wird, soweit wie vorliegend mit einem optischen System gemessen wird, welches einen feststehenden Raumwinkel erfasst.

Bezugszeichenliste:

| | |
|------|------|
| 1 | Beleuchtungssystem |
| 10 | Laser-Lichtquelle |
| 20 | Stecker |
| 30 | Lichtleiter |
| 31 | Kern |
| 31.1 | Kerndurchmesser DC oder Faserbündeldurchmesser |
| 32 | Mantel |
| 40 | Diffusor-Element |
| 41 | Lichteinkopplung |
| 42 | Lichtauskopplung |
| 43 | Diffusor-Grundkörper |
| 43.1 | Durchmesser DD |
| 43.2 | Längsachse |
| 43.3 | Diffusorlänge LD |
| 44 | Verbindungszone |
| 45 | Zwischenmedium |

(fortgesetzt)

| 46 | Hülle |
|---|---|
| 47 | Ballon |
| 48.1 | Flüssigkeitszulauf |
| 48.2 | Flüssigkeitsablauf |
| 49 | Ummantelung |
| 50 | Gewebe |
| 60 | Tumorgewebe |
| 100 | Verlaufsdiagramm |
| 101 | Intensität |
| 102 | Beobachtungswinkel |
| 103.1 | 1. Intensitätsverlauf |
| 103.2 | 2. Intensitätsverlauf |
| 103.3 | 3. Intensitätsverlauf |
| 104 | Intensitätstoleranz |
| 105 | Abstand zur Verbindungszone/Einkoppelstelle |
| 106.1 | 1. Intensitätsverlauf |
| 106.2 | 2. Intensitätsverlauf |
| 107.1 | Spezifische Streulänge LS1 |
| 107.2 | Spezifische Streulänge LS2 |
| 108 | Detektor |

**Patentansprüche**

1. Beleuchtungssystem (1) insbesondere für ein medizintechnisches Therapie- und/oder Diagnosesystem, umfassend wenigstens eine Laser-Lichtquelle (10) und einen Lichtleiter (30), der an einem proximalen Ende an die wenigstens eine Laser-Lichtquelle (10) anschließbar und/oder dieser zuordenbar ist, und welches am distalen Ende des Lichtleiters (30) ein Diffusor-Element (40) aufweist, welches im Wesentlichen eine radiale, sphärische Abstrahlcharakteristik aufweist, wobei das Diffusor-Element (40) mindestens einen Diffusor-Grundkörper (43) umfasst und der Diffusor-Grundkörper (43) ein anorganisches Material, insbesondere ein Glas, eine Glaskeramik, einen glasähnlichen Werkstoff oder einen Verbundwerkstoff aus den zuvor genannten Werkstoffen und zumindest ein Streuelement (43.6) umfasst, wobei die Oberfläche des zumindest einen Diffusor-Grundkörpers (43) einer feuerpolierten Oberflächenqualität entspricht; **gekennzeichnet dadurch, dass** die Lichteinkopplung über eine Einkoppelfläche erfolgt, welche durch eine Verbindungszone des Diffusor-Grundkörpers ausgebildet ist.

2. Beleuchtungssystem (1) nach dem vorstehenden Anspruch, wobei der zumindest eine Diffusor-Grundkörper (43) eine Hülle (46) umfasst, die diesen zumindest teil- oder abschnittsweise oder vollständig umschließt, wobei vorzugsweise die Hülle (46) zumindest die Verbindungszone zwischen Diffusor-Grundkörper (43) und Lichtleiter (30) umschließt.

3. Beleuchtungssystem (1) nach einem der vorstehenden Ansprüche, wobei die Intensitätsverteilung der Abstrahlung um höchstens ± 30 % und bevorzugt höchstens als ± 20 % von einem Durchschnittswert abweicht.

4. Beleuchtungssystem (1) nach einem der vorstehenden Ansprüche, wobei der mindestens eine Diffusor-Grundkörper (43) eine im Wesentlichen kugelförmige, elliptische, tropfenförmige, zylindrische Geometrie oder auch aus diesen Grundformen zusammengesetzte Geometrie aufweist.

5. Beleuchtungssystem (1) nach einem der vorstehenden Ansprüche, wobei der Lichtleiter (30) eine Einzelfaser mit einem Kern (31) mit einem Kerndurchmesser (31.1) und einem Mantel (32) umfasst, wobei der Durchmesser DD (43.1) des Diffusor-Grundkörpers (43) im Bereich der Einkoppelfläche größer oder zumindest gleich groß ist wie der Kerndurchmesser (31.1) des Lichtleiters (30) im Bereich der Einkoppelfläche, wobei vorzugsweise das Verhältnis von Kerndurchmesser (31.1) des Lichtleiters und Durchmesser des Diffusor-Grundkörpers (43) ≤ 1,0 bis 0,3, besonders bevorzugt von ≤ 1,0 bis 0,5 beträgt.

6. Beleuchtungssystem (1) nach einem der vorstehenden Ansprüche, wobei der Lichtleiter (30) ein flexibles Faserbündel oder einen starren Faserstab (31) mit einem Faserbündeldurchmesser oder Faserstabdurchmesser (31.1) umfasst, wobei der Durchmesser DD (43.1) des Diffusor-Grundkörpers (43) im Bereich der Einkoppelfläche größer ist als oder gleich groß wie der Faserbündeldurchmesser (31.1) des Lichtleiters (30) im Bereich der Einkoppelfläche, und wobei vorzugsweise das Verhältnis von Faserbündeldurchmesser oder Faserstabdurchmesser (31.1) des Lichtleiters und Durchmesser des Diffusor-Grundkörpers (43) ≤ 1,0 bis 0,3, besonders bevorzugt von ≤ 1,0 bis 0,5 beträgt.

7. Beleuchtungssystem (1) nach mindestens einem der vorhergehenden Ansprüche, wobei der mindestens eine Diffusor-Grundkörper (43) und der Lichtleiter (30) mittels Kleben oder Spleißen fest miteinander verbunden sind, und/oder wobei sich zwischen dem Diffusor-Grundkörper (43) und dem distalen Ende des Lichtleiters (30) eine Verbindungszone (44) befindet, in der ein optisches Element und/oder ein Zwischenmedium (45) angeordnet ist.

8. Beleuchtungssystem (1) nach mindestens einem der vorhergehenden Ansprüche, wobei die Hülle (46) eine für seitlich aus dem Diffusor-Element emittiertes Licht transparente oder transluzente Schicht, aufweisend organischen Lack, Flüssig-Silikon, thermoplastischen Polymer, Schmelzkleber, 2-komponentigen Kleber oder Sol-Gel-Glas, einen Schrumpfschlauch oder zusätzlich aufgesetzte transparente oder transluzente Aufsatzelemente umfasst, wobei vorzugsweise die Hülle (46) ein niedrig schmelzendes Glas umfasst, welches eine niedrige Verarbeitungstemperatur von vorzugsweise kleiner 500 °C, bevorzugt kleiner als 400 °C und besonders bevorzugt kleiner als 300 °C aufweist und/oder wobei die Verarbeitungstemperatur des aufzubringenden Glases um wenigstens 50 K, bevorzugt wenigstens 100 K niedriger als die Erweichungstemperatur des Diffusor-Elements ist, und/oder wobei die Hülle (46) aus einer transparenten oder lichtstreuenden Aufsatzkappe aus Kunststoff oder Glas ausgeführt ist.

9. Beleuchtungssystem (1) nach einem der vorstehenden Ansprüche, wobei der Diffusor-Grundkörper Streuzentren umfasst in Form von Poren, Partikel, Kristallite, Polykristallite, poröse und/oder pigmentierte und/oder optisch aktive Pigmente, insbesondere Leuchtstoffe mit Phosphoreszenz oder Fluoreszenz und/oder eingefärbte Bereiche, beispielsweise eingefärbte Partikel, eingefärbte Kristallite oder eingefärbte Pigmente, oder Färbungen des Glases oder Inhomogenitäten in Form von Brechungsindexschwankungen oder beliebige Kombinationen derartiger Streuzentren, wobei die Inhomogenitäten des anorganischen Materials Phasenseparation, Entmischungen und/oder partikuläre Einlagerung, Keime und/oder Kristallite umfassen, wobei vorzugsweise die Konzentration der Streuzentren im Streubereich zwischen 10 ppm bis 1.000 ppm, bevorzugt zwischen 20 ppm bis 100 ppm beträgt.

10. Beleuchtungssystem (1), nach mindestens einem der vorherigen Ansprüche, wobei der Diffusor-Grundkörper (43) eine homogene Verteilung von Streuzentren über sin Gesamtvolumen aufweist, und/oder wobei die Streuzentren einen Durchmesser bzw. eine maximale Ausdehnung in einer Richtung von 10 nm bis 5.000 nm auf, besonders bevorzugt von 100 nm bis 1.200 nm aufweisen.

11. Beleuchtungssystem (1) nach einem der vorstehenden Ansprüche, wobei die Streupartikel $SiO_2$ und/oder SiN und/oder BaO und/oder MgO und/oder ZnO und/oder $Al_2O_3$ und/oder AlN und/oder $TiO_2$ und/oder $ZrO_2$ und/oder $Y_2O_3$ und/oder die Metalle dieser Oxide alleine und/oder BN und/oder $B_2O_3$ und/oder Ru und/oder Os und/oder Rh und/oder Ir und/oder Ag und/oder Au und/oder Pd und/oder Pt und/oder diamantartigem Kohlenstoff und/oder Glaskeramik-Partikel umfassen.

12. Beleuchtungssystem (1) nach einem der vorstehenden Ansprüche, wobei der mindestens eine Diffusor-Grundkörper (43) ein Silikatglas, bevorzugt en Na-Al-K-Silikatglas und ganz besonders bevorzugt ein Na-Al-K-Ca-Zn-Silikatglas oder ein Na-Al-K-As-Pb-Silikatglas umfasst,

oder wobei der mindestens eine Diffusor-Grundkörper (43) ein Borosilikatglas umfasst, vorzugsweise mit folgender Zusammensetzung (in Gew.-%):

$B_2O_3$     15,00-40,00

(fortgesetzt)

| | |
|---|---|
| $SiO_2$ | 45,00-80,00 |
| $R_2O$ | 0,0-20,0 |
| R'O | 0,0-20,00 |
| $R''O_2$ | 0,0-10,00 |
| $Al_2O_3$ | 0,0-10,00, |

oder wobei der mindestens eine Diffusor-Grundkörper (43) eine Glaskeramik umfasst, bevorzugt eine Lithium-Alumo-Silikat-Glaskeramik (LAS-GK), eine Cordierit-Glaskeramik oder eine Magnesium-Aluminium-Silikat Glaskeramik, besonders bevorzugt eine klar transparenten Lithium-Alumo-Silikat-Glaskeramik, welche durch eine gezielte Temperatur-/Zeitbeaufschlagung zumindest partiell in einen Zustand mit lichtstreuenden Kristalliten umgewandelt ist.

13. Beleuchtungssystem (1), nach einem der vorhergehenden Ansprüche, wobei das Diffusor-Element (40) und/oder der Diffusor-Grundkörper (43) und/oder die Hülle (46) eine niedrige Absorption bei der jeweiligen Anwendungswellenlänge aufweist,

und/oder wobei das Diffusor-Element (40) und/oder der Diffusor-Grundkörper (43) und/oder die Hülle (46) zumindest teilweise oder abschnittsweise in deren Volumen und/oder auf deren Oberflächen strukturiert ist, und/oder wobei der Diffusor-Grundkörper (43) und/oder die Hülle (46) eine Beschichtung aus streuenden Partikeln und/oder der Diffusor-Grundkörper (43) eine zusätzliche Ummantelung aus einem eingefärbten Glas oder einem eingefärbten Kunststoff aufweist,
und/oder wobei das Diffusor-Element (40) und/oder der Diffusor-Grundkörper (43) und/oder die Hülle von einem aufpumpbaren transparenten oder lichtstreuenden Ballon (47) umhüllt ist,
und/oder wobei das Diffusor-Element (40) und/oder der Diffusor-Grundkörper (43) im Ballon (47) von einer Kühlflüssigkeit oder einem Gas umspülbar ist und neben dem Lichtleiter (30) zusätzliche Zu- und Ablaufkanäle für diese Kühlflüssigkeit vorgesehen sind.

14. Verfahren zum Herstellen eines Diffusor-Grundkörpers (43), insbesondere für ein Beleuchtungssystem (1), nach einem der vorhergehenden Ansprüche, umfassend die folgenden Schritte:

- Bereitstellen einer Glasschmelze aus Glasrohstoffen einer vorbestimmten Zusammensetzung zur Erzeugung eines Ausgangsglases, vorzugsweise Borosilikatglas, bevorzugt Alkali-Borosilikatglas,
- Durchführen einer thermischen Behandlung mit kontrolliertem Temperatur-/Zeitverlauf zur Phasentrennung in dem Temperaturbereich von 500-800 °C, beispielsweise 600-800 °C, bevorzugt 650-750 °C, besonders bevorzugt 700-725 °C,
- für eine Zeitspanne von 1 bis 150 Stunden, beispielsweise 24 bis 48 Stunden oder 48 bis 80 Stunden,
- Erzeugen von Streuzentren mittels dieser thermischen Behandlung in dem Material, wobei sich die Zusammensetzung in eine silikatreiche Phase und eine borreiche Phase trennt, zum Erzeugen einer offenen Porosität innerhalb des Glases durch einen Säurelaugungsschritt und/oder einen Porenreinigenden kaustischen Laugungsschritt, um ein Glas mit disperse Phase-Partikeln oder Poren in der Größenordnung von 200 bis 700 nm, z.B. 200 bis 500 nm, bevorzugt 300-500 nm, besonders bevorzugt 300-450 nm, mit einer Anzahldichte von annähernd $10^8$ bis $10^{12}$ mm$^{-3}$, bevorzugt $10^9$ bis $10^{11}$ mm$^{-3}$, besonders bevorzugt $10^{10}$ bis $10^{11}$ mm$^{-3}$, zu erhalten, wobei der auf diese Weise erhaltene Diffusor-Grundkörper mit einem Lichtleiter mittels Kleben oder Spleißen fest miteinander verbunden wird, **gekennzeichnet dadurch, dass** der Diffusor-Grundkörper und/oder der mit dem Lichtleiter verbundene Diffusor-Grundkörper zumindest einschließend die Verbindungszone zwischen Diffusor-Grundkörper und Lichtleiter einem Beschichtungsprozess unterzogen werden, wodurch eine Hülle (46) ausgebildet wird zum Schutz des Diffusor-Grundkörpers.

15. Verfahren zum Herstellen eines Diffusor-Grundkörpers (43) nach vorstehendem Anspruch, wobei mittels der Temperatur-/Zeit-Führung eine wellenlängenabhängige Streuwirkung erzielt wird.

16. Vorrichtung für ein medizinisches Therapieverfahren, insbesondere für eine photodynamische Therapie (PDT) und/oder Photo-Immuno-Therapie (PIT) zur Tumortherapie, für eine endovenöse Lasertherapie (EVLT) zur Behandlung von Krampfadern, für eine Blasen- oder Prostata-Behandlung, für eine Laser- induzierte interstitielle Thermotherapie (LITT), für eine lichtinduzierte Behandlung von Entzündungen im Hals-/Rachenraum (Mukositis) oder für Anwendungen im Bereich der Dentalmedizin, Augenheilkunde (Ophtalmologie) sowie Dermatologie, umfassend ein Beleuchtungssystem (1) nach einem der vorstehenden Ansprüche 1 bis 13.

**17.** Vorrichtung für eine photodynamische Therapie (PDT) zur Tumortherapie, wobei zumindest ein Lichtleiter (30) mit dem Diffusor-Element (40) aus anderen Diffusor-Elementen (40) abgestrahltes Licht aufnimmt und über den Lichtleiter (30) einem Detektor zur spektroskopischen Analyse und/oder zur Dosimetrie weiterleitet, umfassend ein Beleuchtungssystem (1) nach einem der vorstehenden Ansprüche 1 bis 13.

## Claims

**1.** A lighting system (1), in particular for a medical technology therapy system and/or diagnostic system, comprising at least one laser light source (10) and an optical waveguide (30), which can be coupled to and/or associated with the at least one laser light source (10) at a proximal end thereof, and comprising a diffuser element (40) at the distal end of the optical waveguide (30), which substantially exhibits a radial, spherical radiation characteristic, wherein the diffuser element (40) comprises at least one diffuser base body (43) and the diffuser base body (43) comprises an inorganic material, in particular a glass, a glass ceramic, a glass-like material or a composite material made from the aforementioned materials and at least one scattering element (43.6), wherein the surface of the at least one diffuser base body (43) corresponds to a fire-polished surface quality;
**characterized in that** light is injected via an injection area which is defined by a coupling area of the diffuser base body.

**2.** The lighting system (1) according to the preceding claim, wherein the at least one diffuser base body (43) comprises a cover (46) enclosing it at least partially or in sections thereof or completely; wherein, preferably, the cover (46) encloses at least the coupling area between the diffuser base body (43) and the optical waveguide (30).

**3.** The lighting system (1) according to any one of the preceding claims, wherein the radiation intensity distribution deviates from a mean value by not more than $\pm$ 30 % and preferably by not more than $\pm$ 20 %.

**4.** The lighting system (1) according to any one of the preceding claims, wherein the at least one diffuser base body (43) has any of a substantially spherical, elliptical, drop-shaped, cylindrical geometry or a geometry composed of these basic shapes.

**5.** The lighting system (1) according to any one of the preceding claims, wherein the optical waveguide (30) comprises a single fibre having a core (31) with a core diameter (31.1) and a cladding (32), wherein in the vicinity of the injection area the diameter DD (43.1) of the diffuser base body (43) is greater than or at least of the same size as the core diameter (31.1) of the optical waveguide (30) in the vicinity of the injection area, wherein preferably the ratio of the core diameter (31.1) of the optical waveguide to the diameter of the diffuser base body (43) ranges from $\leq$ 1.0 to 0.3, most preferably from $\leq$ 1.0 to 0.5.

**6.** The lighting system (1) according to any one of the preceding claims, wherein the optical waveguide (30) comprises a flexible fibre bundle or a rigid fibre rod (31) having a fibre bundle diameter or a fibre rod diameter (31.1), wherein in the vicinity of the injection area the diameter DD (43.1) of the diffuser base body (43) is greater than or equal to the fibre bundle diameter (31.1) of the optical waveguide (30) in the vicinity of the injection area, and wherein preferably the ratio of the fibre bundle diameter or fibre rod diameter (31.1) of the optical waveguide to the diameter of the diffuser base body (43) ranges from $\leq$ 1.0 to 0.3, most preferably from $\leq$ 1.0 to 0.5.

**7.** The lighting system (1) according to at least one of the preceding claims, wherein the at least one diffuser base body (43) and the optical waveguide (30) are firmly connected to one another by gluing or splicing; and/or wherein a coupling zone (44) is provided between the diffuser base body (43) and the distal end of the optical waveguide (30), in which an optical element and/or an intermediate medium (45) is arranged.

**8.** The lighting system (1) according to at least one of the preceding claims, wherein the cover (46) comprises a layer which is transparent or translucent for light emitted laterally from the diffuser element, comprising any of an organic lacquer, liquid silicone, thermoplastic polymer, hot melt adhesive, 2-component adhesive or sol-gel glass, a shrink tube or additionally attached transparent or translucent attachment elements; wherein, preferably, the cover (46) comprises a low-melting glass having a low processing temperature of preferably less than 500 °C, more preferably less than 400 °C and most preferably less than 300 °C;

and/or wherein the processing temperature of the glass to be applied is lower than the softening temperature of the diffuser element by at least 50 K, preferably by at least 100 K;
and/or wherein the cover (46) is made of a transparent or light-scattering attachment cap made of plastics material or of glass.

9. The lighting system (1) according to any one of the preceding claims, wherein the diffuser base body comprises scattering centres in the form of pores, particles, crystallites, polycrystallites, porous and/or pigmented and/or optically active pigments, in particular luminescent materials with phosphorescence or fluorescence and/or dyed areas, for example dyed particles, dyed crystallites or dyed pigments, or colorations of the glass, or inhomogeneities in the form of variations in the refractive index or any combination of such scattering centres, wherein the inhomogeneities of the inorganic material comprise phase separation, demixing and/or particulate inclusions, nuclei and/or crystallites, wherein the concentration of the scattering centres in the scattering area is preferably between 10 ppm and 1,000 ppm, more preferably between 20 ppm and 100 ppm.

10. The lighting system (1) according to at least one of the preceding claims, wherein the diffuser base body (43) exhibits a homogeneous distribution of scattering centres throughout its volume; and/or wherein the scattering centres have a diameter or a maximum extent in one direction of 10 nm to 5,000 nm, most preferably of 100 nm to 1,200 nm.

11. The lighting system (1) according to any one of the preceding claims, wherein the scattering particles comprise $SiO_2$ and/or SiN and/or BaO and/or MgO and/or ZnO and/or $Al_2O_3$ and/or AlN and/or $TiO_2$ and/or $ZrO_2$ and/or $Y_2O_3$ and/or the metals of these oxides alone and/or BN and/or $B_2O_3$ and/or Ru and/or Os and/or Rh and/or Ir and/or Ag and/or Au and/or Pd and/or Pt and/or diamond-like carbon and/or glass-ceramic particles.

12. The lighting system (1) according to any one of the preceding claims,

wherein the at least one diffuser base body (43) comprises a silicate glass, preferably a Na-Al-K silicate glass, and most preferably a Na-Al-K-Ca-Zn silicate glass or a Na-Al-K-As-Pb silicate glass;
or wherein the at least one diffuser base body (43) comprises a borosilicate glass,
preferably with the following composition (in wt%):

| | |
|---|---|
| $B_2O_3$ | 15.00 - 40.00 |
| $SiO_2$ | 45.00 - 80.00 |
| $R_2O$ | 0.0 - 20.0 |
| R'O | 0.0 - 20.00 |
| $R''O_2$ | 0.0 - 10.00 |
| $Al_2O_3$ | 0.0 - 10.00; |

or wherein the at least one diffuser base body (43) comprises a glass ceramic,
preferably a lithium aluminosilicate glass ceramic (LAS GC), a cordierite glass ceramic or a magnesium aluminium silicate glass ceramic, most preferably a clear transparent lithium aluminosilicate glass ceramic which, through selective temperature-time exposure, has been at least partially converted into a state including light-scattering crystallites.

13. The lighting system (1) according to any one of the preceding claims,

wherein the diffuser element (40) and/or the diffuser base body (43) and/or the cover (46) exhibits low absorption at the respective application wavelength;
and/or wherein the diffuser element (40) and/or the diffuser base body (43) and/or the cover (46) is textured at least partially or in sections thereof in the bulk and/or on the surfaces thereof;
and/or wherein the diffuser base body (43) and/or the cover (46) has a coating of scattering particles and/or wherein the diffuser base body (43) has an additional jacket made of a dyed glass or a dyed plastics material;
and/or wherein the diffuser element (40) and/or the diffuser base body (43) and/or the cover is covered by an inflatable transparent or light-scattering balloon (47); and/or wherein the diffuser element (40) and/or the diffuser base body (43) can be flushed by a cooling liquid or a gas inside the balloon (47) and additional inlet and outlet passages are provided for this cooling liquid adjacent to the optical waveguide (30).

14. A method for producing a diffuser base body (43), in particular for a lighting system (1) according to any one of the preceding claims, comprising the steps of:

- providing a glass melt from glass raw materials of a predefined composition for producing a starting glass, preferably a borosilicate glass, more preferably an alkali borosilicate glass;
- performing a thermal treatment with controlled temperature-time profile for phase separation in the temperature range 500 - 800 °C, for example 600 - 800 °C, preferably 650 - 750 °C, most preferably 700 - 725 °C;
- over a duration of 1 to 150 hours, for example 24 to 48 hours or 48 to 80 hours;
- generating scattering centres by virtue of this thermal treatment in the material, whereby the composition separates into a silicate-rich phase and a boron-rich phase, for generating open porosity within the glass through an acid leaching step and/or a pore cleaning caustic leaching step, in order to obtain a glass having disperse phase particles or pores in the order of 200 to 700 nm, e.g. 200 to 500 nm, preferably 300 - 500 nm, most preferably 300 - 450 nm, with a number density of approximately $10^8$ to $10^{12}$ $mm^{-3}$, preferably $10^9$ to $10^{11}$ $mm^{-3}$, most preferably $10^{10}$ to $10^{11}$ $mm^{-3}$, wherein the diffuser base body obtained in this way is firmly connected to an optical waveguide by gluing or splicing; **characterized in that** the diffuser base body and/or the optical waveguide connected to the optical waveguide, at least including the coupling area between the diffuser base body and the optical waveguide, are subjected to a coating process, whereby a cover (46) is formed for protecting the diffuser base body.

15. The method for producing a diffuser base body (43) according to the preceding claim, wherein a wavelength-dependent scattering effect is achieved through the temperature-time control.

16. A device for a medical therapy procedure, in particular for photodynamic therapy (PDT) and/or photoimmunotherapy (PIT) for tumour therapy, for endovenous laser therapy (EVLT) for the treatment of varicose veins, for bladder or prostate treatment, for laser-induced interstitial thermotherapy (LITT), for light-induced treatment of inflammations in the throat/pharynx (mucositis), or for applications in the field of dentistry, eye care (ophthalmology) and dermatology, comprising a lighting system (1) according to any one of the preceding claims 1 to 13.

17. A photodynamic therapy (PDT) device for tumour therapy, wherein at least one optical waveguide (30) having the diffuser element (40) receives light emitted from other diffuser elements (40) and transmits it through the optical waveguide (30) to a detector for spectroscopic analysis and/or for dosimetry, comprising a lighting system (1) according to any one of the preceding claims 1 to 13.

**Revendications**

1. Système d'éclairage (1), destiné en particulier à un système technique médical de thérapie et/ou de diagnostic, comprenant au moins une source de lumière laser (10) et un guide de lumière (30) qui peut être raccordé par une extrémité proximale à la source de lumière laser (10), au nombre d'au moins une, et/ou peut être associé à celle-ci, et qui comporte, à l'extrémité distale du guide de lumière (30), un élément diffuseur (40) présentant sensiblement une caractéristique de rayonnement sphérique radiale, l'élément diffuseur (40) comprenant au moins un corps de base de diffuseur (43), et le corps de base de diffuseur (43) comportant un matériau inorganique, notamment un verre, une vitrocéramique, un matériau similaire au verre ou un matériau composite constitué des matériaux précités, et comprenant au moins un élément de diffusion (43.6), la surface du corps de base de diffuseur (43), au nombre d'au moins un, correspondant à une qualité de surface polie au feu, **caractérisé en ce que** le couplage de la lumière s'effectue par l'intermédiaire d'une surface de couplage qui est constituée d'une zone de liaison du corps de base de diffuseur (43).

2. Système d'éclairage (1) selon la revendication précédente, dans lequel le corps de base de diffuseur (43), au nombre d'au moins un, comprend une enveloppe (46) qui entoure celui-ci au moins en partie ou par portions ou complètement, l'enveloppe (46) entourant de préférence au moins la zone de liaison entre le corps de base de diffuseur (43) et le guide de lumière (30).

3. Système d'éclairage (1) selon une des revendications précédentes, dans lequel la répartition d'intensité du rayonnement présente un écart d'au maximum + 30 % et de préférence d'au maximum + 20 % de la valeur moyenne.
3. Système d'éclairage (1) selon une des revendications précédentes, dans lequel la répartition d'intensité du rayonnement présente un écart d'au maximum + 30 % et de préférence d'au maximum + 20 % par rapport à une valeur moyenne.

**4.** Système d'éclairage (1) selon une des revendications précédentes, dans lequel le corps de base de diffuseur (43), au nombre d'au moins un, présente une géométrie sensiblement sphérique, elliptique, en forme de goutte ou cylindrique ou bien également une géométrie composée de ces formes de base.

**5.** Système d'éclairage (1) selon une des revendications précédentes, dans lequel le guide de lumière (30) comprend une fibre individuelle comportant un coeur (31), avec un diamètre de coeur (31.1), et une gaine (32), le diamètre DD (43.1) du corps de base de diffuseur (43), dans la région de la surface de couplage, étant supérieur ou au moins égal au diamètre de coeur (31.1) du guide de lumière (30) dans la région de la surface de couplage, le rapport entre le diamètre de coeur (31.1) du guide de lumière et le diamètre du corps de base de diffuseur (43) étant de préférence ≤ 1,0 à 0,3, et de manière particulièrement avantageuse ≤ 1,0 à 0,5.

**6.** Système d'éclairage (1) selon une des revendications précédentes, dans lequel le guide de lumière (30) comprend un faisceau de fibres souple ou une tige de fibres (31) rigide présentant un diamètre de faisceau de fibres ou un diamètre de tige de fibres (31.1), le diamètre DD (43.1) du corps de base de diffuseur (43), dans la région de la surface de couplage, étant supérieur ou égal au diamètre de faisceau de fibres (31.1) du guide de lumière (30) dans la région de la surface de couplage, et le rapport entre le diamètre de faisceau de fibres ou le diamètre de tige de fibres (31.1) du guide de lumière et le diamètre du corps de base de diffuseur (43) étant de préférence ≤ 1,0 à 0,3, et de manière particulièrement avantageuse ≤ 1,0 à 0,5.

**7.** Système d'éclairage (1) selon au moins une des revendications précédentes, dans lequel le corps de base de diffuseur (43), au nombre d'au moins un, et le guide de lumière (30) sont liés solidement l'un à l'autre par collage ou épissage, et/ou dans lequel il est prévu entre le corps de base de diffuseur (43) et l'extrémité distale du guide de lumière (30), une zone de liaison (44) dans laquelle est disposé un élément optique et/ou un milieu intermédiaire (45).

**8.** Système d'éclairage (1) selon au moins une des revendications précédentes, dans lequel l'enveloppe (46) comprend une couche qui est transparente ou translucide pour la lumière émise latéralement depuis l'élément diffuseur et qui présente une peinture organique, du silicone liquide, un polymère thermoplastique, un adhésif thermo-fusible, un adhésif à deux composants ou un verre sol-gel, un tube ther-morétractable ou des éléments rapportés transparents ou translucides supplémentaires, l'enveloppe (46) comprenant de préférence un verre à bas point de fusion qui présente une faible température de mise en oeuvre, de préférence inférieure à 500 °C, notamment inférieure à 300 °C, et/ou la température de mise en oeuvre du verre à appliquer étant de préférence inférieure d'au moins 50 K, de préférence d'au moins 100 K à la température de ramollissement de l'élément diffuseur, et/ou l'enveloppe (46) étant réalisée à partir d'un capuchon rapporté transparent ou diffusant la lumière, constitué de matière plastique ou de verre.

**9.** Système d'éclairage (1) selon une des revendications précédentes, dans lequel le corps de base de diffuseur (43) comprend des centres diffuseurs sous forme de pores, de particules, de cristallites, de polycristallites, de pigments poreux et/ou pigmentés et/ou optiquement actifs, notamment des matières luminescentes à phosphorescence ou à fluorescence, et/ou des zones teintées, par exemple des particules teintées, des cristallites teintés ou des pigments teintés, ou bien des teintures du verre ou des inhomogénéités sous forme de variations de l'indice de réfraction, ou des combinaisons quelconques de centres diffuseurs de ce type, les inhomogénéités du matériau inorganique englobant la séparation de phase, des ségrégations et/ou une incorporation particulaire, des germes et/ou des cristallites, la concentration des centres diffuseurs dans la zone de diffusion étant de préférence comprise entre 10 ppm et 1 000 ppm, de préférence entre 20 ppm et 100 ppm.

**10.** Système d'éclairage (1) selon au moins une des revendications précédentes, dans lequel le corps de base de diffuseur (43) présente une répartition homogène de centres diffuseurs dans son volume total, et/ou dans lequel les centres diffuseurs présentent un diamètre ou une dimension maximale dans une direction qui sont compris entre 10 nm et 5 000 nm, et de manière particulièrement avantageuse entre 100 nm et 1 200 nm.

**11.** Système d'éclairage (1) selon une des revendications précédentes, dans lequel les particules de diffusion comportent du $SiO_2$ et/ou SiN et/ou BaO et/ou MgO et/ou ZnO et/ou $Al_2O_3$ et/ou AIN et/ou $TiO_2$ et/ou $ZrO_2$ et/ou $Y_2O_3$ et/ou les métaux de ces oxydes seuls et/ou BN et/ou $B_2O_3$ et/ou Ru et/ou Os et/ou Rh et/ou Ir et/ou Ag et/ou Au et/ou Pd et/ou Pt et/ou du carbone de type diamant et/ou des particules de vitrocéramique.

**12.** Système d'éclairage (1) selon une des revendications précédentes, dans lequel le corps de base de diffuseur (43), au nombre d'au moins un, comprend un verre de silicate, de préférence un verre de silicate Na-Al-K et de manière particulièrement avantageuse un verre de silicate Na-Al-K-Ca-Zn ou un verre de silicate Na-Al-K-As-Pb,

ou dans lequel le corps de base de diffuseur (43), au nombre d'au moins un, comprend un verre de borosilicate ayant de préférence la composition suivante (en % en poids) :

| | |
|---|---|
| $B_2O_3$ | 15,00 à 40,00 |
| $SiO_2$ | 45,00 à 80,00 |
| $R_2O$ | 0,00 à 20,00 |
| R'O | 0,00 à 20,00 |
| $R"O_2$ | 0,00 à 10,00 |
| $Al_2O_3$ | 0,00 à 10,00, |

ou dans lequel le corps de base de diffuseur (43), au nombre d'au moins un, comprend une vitrocéramique, de préférence une vitrocéramique en aluminosilicate de lithium (LAS), une vitrocéramique en cordiérite ou une vitrocéramique en aluminosilicate de magnésium, de manière particulièrement avantageuse une vitrocéramique en aluminosilicate de lithium transparente claire qui est amenée au moins partiellement à un état comportant des cristallites diffuseurs de lumière par une sollicitation ciblée de température/temps.

13. Système d'éclairage (1) selon une des revendications précédentes, dans lequel l'élément diffuseur (40) et/ou le corps de base de diffuseur (43) et/ou l'enveloppe (46) présentent une faible absorption en présence de la longueur d'onde d'application respective,

et/ou dans lequel l'élément diffuseur (40) et/ou le corps de base de diffuseur (43) et/ou l'enveloppe (46) sont structurés au moins partiellement ou par portions dans leur volume et/ou à leurs surfaces,
et/ou dans lequel le corps de base de diffuseur (43) et/ou l'enveloppe (46) présentent un revêtement de particules diffusantes et/ou le corps de base de diffuseur (43) présente un enrobage supplémentaire fait d'un verre teinté ou d'une matière plastique teintée,
et/ou dans lequel l'élément diffuseur (40) et/ou le corps de base de diffuseur (43) et/ou la gaine sont entourés d'un ballon (47) gonflable transparent ou diffuseur de lumière,
et/ou dans lequel l'élément diffuseur (40) et/ou le corps de base de diffuseur (43) peuvent être baignés dans le ballon (47) dans un fluide de refroidissement ou un gaz, et que des conduits d'arrivée et d'évacuation supplémentaires pour ce liquide de refroidissement sont prévus à côté du guide de lumière (30).

14. Procédé de fabrication d'un corps de base de diffuseur (43), en particulier pour un système d'éclairage (1) selon une des revendications précédentes, comprenant les étapes suivantes :

- préparation d'un verre en fusion à partir de matières brutes de verre ayant une composition prédéfinie, en vue de la fabrication d'un verre de départ, de préférence de verre borosilicate, notamment de verre borosilicate alcalin,
- réalisation d'un traitement thermique avec une évolution température/temps contrôlée, en vue de la séparation de phases dans la plage de températures allant de 500 à 800 °C, par exemple de 600 à 800 °C, de préférence de 650 à 750 °C, et de manière particulièrement avantageuse de 700 à 725 °C,
- pendant un laps de temps allant de 1 à 150 heures, par exemple de 24 à 48 heures ou de 48 à 80 heures,
- création de centres diffuseurs dans le matériau à l'aide de ce traitement thermique, le composé se séparant en une phase riche en silicate et une phase riche en bore, en vue de la création d'une porosité ouverte dans le verre, par une étape de lixiviation à l'acide et/ou une étape de lixiviation caustique de nettoyage des pores, aux fins d'obtenir un verre comportant des particules de phase dispersée ou des pores d'un ordre de grandeur allant de 200 à 700 nm, par exemple de 200 à 500 nm, de préférence de 300 à 500 nm, de manière particulièrement avantageuse de 300 à 450 nm, avec une densité du nombre de près de $10^8$ à $10^{12}$ mm$^{-3}$, de préférence de $10^9$ à $10^{11}$ mm$^{-3}$, de manière particulièrement avantageuse de $10^{10}$ à $10^{11}$ mm$^{-3}$, le corps de base de diffuseur ainsi obtenu étant relié solidement à un guide de lumière par collage ou épissage, **caractérisé en ce que** le corps de base de diffuseur et/ou le corps de base de diffuseur relié au guide de lumière sont soumis à un processus de revêtement, en incluant au moins la zone de liaison entre le corps de base de diffuseur et le guide de lumière, ce qui a pour effet de former une enveloppe (46) pour protéger le corps de base de diffuseur.

15. Procédé de fabrication d'un corps de base de diffuseur (43) selon la revendication précédente, selon lequel la gestion température-temps permet d'obtenir un effet de dispersion dépendant de la longueur d'onde.

16. Dispositif destiné à une méthode thérapeutique médicale, en particulier une thérapie photodynamique (PDT) et/ou une photo-immunothérapie (PIT) de traitement de tumeurs, une thérapie au laser endoveineux (EVLT) de traitement de varices, un traitement de la vessie ou de la prostate, une thermothérapie interstitielle induite par laser (LITT), un traitement induit par la lumière d'inflammations dans la région de la gorge/du pharynx (mucite) ou des applications dans le domaine de la médecine dentaire, de l'ophtalmologie et de la dermatologie, comprenant un système d'éclairage (1) selon une des revendications précédentes 1 à 13.

17. Dispositif destiné à une thérapie photodynamique (PDT) de traitement de tumeurs, selon lequel au moins un guide de lumière (30) reçoit avec l'élément diffuseur (40) de la lumière rayonnée par d'autres éléments diffuseurs (40) et la transmet par l'intermédiaire du guide de lumière (30) à un détecteur en vue de l'analyse spectroscopique et/ou de la dosimétrie, comprenant un système d'éclairage (1) selon une des revendications précédentes 1 à 13.

Fig. 1

Fig. 2a          Fig. 2b          Fig. 2c

EP 3 897 827 B1

Fig. 3

Fig. 4

104   103.1   103.2   101                    100

-90°              0°              +90°

102

Fig. 5

40                    47

30

49

48.2                    48.1

Fig. 6

Fig. 7a          Fig. 7b          Fig. 7c

Fig. 8

Fig. 9

Fig. 10

Fig. 11

Fig. 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10129029 A1
- EP 3184885 A1
- US 6810184 B2
- EP 2062077 A4
- US 20090204111 A1
- DE 102015119875 A1
- WO 2008024397 A2
- WO 2017103796 A1

- EP 2018076487 W
- DE 102013208838 A1
- WO 2009100834 A
- WO 2014165048 A1
- DE 112014001293 T5
- US 20130017387 A, James
- EP 1266543 A1

**In der Beschreibung aufgeführte Nicht-Patentliteratur**

- Photodynamic Therapy of Cancer. *Cancer Medicine,* 2003
- **KAWAMOTO ; TOMOZAWA.** *J. Amer. Ceram. Soc.,* 1981, vol. 64 (5), 289-292
- **ZDANIEWSKI.** *J. Amer. Ceram. Soc.,* 1978, vol. 61 (5-6), 199-204

- **RANDALL et al.** *J. Amer. Ceram. Soc.,* 1988, vol. 71 (12), 1134-1141
- **FINLAY et al.** *Proc. SPIE Int. Soc. Opt. Eng.,* 14. Juni 2014, vol. 5315, 132-142